(19) Europäisches Patentamt

European Patent Office

Office européen des brevets

(11) EP 0 783 003 B1

(12) EUROPEAN PATENT SPECIFICATION

(45) Date of publication and mention
of the grant of the patent:
**22.11.2006 Bulletin 2006/47**

(51) Int Cl.:
*C07K 19/00* (2006.01)   *C07K 14/535* (2006.01)
*C07K 14/52* (2006.01)   *C12N 15/62* (2006.01)
*A61K 38/19* (2006.01)   *C12P 21/02* (2006.01)
*C12N 5/10* (2006.01)

(21) Application number: 96912262.1

(22) Date of filing: **26.04.1996**

(86) International application number:
**PCT/JP1996/001157**

(87) International publication number:
**WO 1996/034016 (31.10.1996 Gazette 1996/48)**

(54) **G-CSF TPO fusion proteins**

G-CSF TPO Fusionsproteine

Proteines hybrides G-CSF TPO

(84) Designated Contracting States:
**AT BE CH DE DK ES FI FR GB GR IE IT LI LU MC
NL PT SE**

(30) Priority: **26.04.1995 JP 10262595**

(43) Date of publication of application:
**09.07.1997 Bulletin 1997/28**

(73) Proprietor: **KYOWA HAKKO KOGYO CO., LTD.
Chiyoda-ku,
Tokyo 100 (JP)**

(72) Inventors:
 • **YOKOI, Haruhiko
  Ibaraki 305 (JP)**
 • **SHIOTSU, Yukimasa
  Tokyo 157 (JP)**
 • **KONISHI, Noboru
  Yamaguchi 747 (JP)**
 • **ANAZAWA, Hideharu
  Tokyo 178 (JP)**
 • **TAMAOKI, Tatsuya
  Tokyo 194 (JP)**

 • **YAMASAKI, Motoo
  Tokyo 194 (JP)**
 • **TERASAKI, Yoko
  Tokyo 194 (JP)**
 • **UCHIDA, Kazuhisa
  Tokyo 194 (JP)**
 • **YAMASHITA, Kinya
  Shizuoka 411 (JP)**

(74) Representative: **Kinzebach, Werner et al
Patentanwälte
Reitstötter, Kinzebach und Partner
Postfach 86 06 49
81633 München (DE)**

(56) References cited:
**WO-A-90/12877         JP-A- 1 316 400
JP-A- 4 103 599        JP-T- 5 502 463
JP-T- 6 500 116**

 • **NATURE, (1994), Vol. 369, DE SAUVAGE F.J. et
al., "Stimulation of Megakaryocytopoiesis and
Thrombopoiesis by the c-Mpl Ligand", pages
533-538.**

**Description**

TECHNICAL FIELD

The present invention relates to a fusion polypeptide comprising a polypeptide having a granulocyte colony stimulating factor (hereinafter referred to as "G-CSF") activity and a polypeptide having a platelet growth factor (thrombopoietin, hereinafter referred to as "TPO") activity, and DNA which codes for the fusion polypeptide. Since the fusion polypeptide of the present invention can form and amplify platelets and neutrophils simultaneously, it is useful for the treatment of anemia and the like.

BACKGROUND ART

Blood comprises hematopoietic cells such as erythrocytes, leukocytes, platelets and the like. These hematopoietic cells mature from only one kind of pluripotential blood stem cell through various differentiation steps. These steps undergo complex regulation by a group of proteinous factors which are generally referred to as cytokines. A certain type of cytokine takes part in the differentiation and multiplication of various hematopoietic cells. On the other hand, a certain type of hematopoietic cell undergoes regulation of its differentiation and multiplication by various types of cytokines. This is called overlapping cytokine actions. Among these cytokine members, TPO and G-CSF are considered to have small overlapping actions.

TPO mainly takes part in the formation of platelets. Platelets are formed by the fragmentation of megakaryocytes, a hematopoietic cell which has large nucleus and is present mainly in bone marrow. Platelets are essential for forming blood clots at damaged portions in blood vessels. Platelets also play important roles in not only blood coagulation but also injury healing by releasing proteins having other functions at the damaged portions. A significant decrease in the number of platelets may be fatal, because the body may easily bleed.

G-CSF is a cytokine which accelerates activation of neutrophils, a member of the leukocytes, and differentiation of neutrophils from their precursor cells. Neutrophils exert the first defense action when invaded by foreign enemies such as bacteria, viruses and the like. When the number of neutrophils is decreased, the body becomes defenseless against infection, and this too is also often fatal.

Current medical treatment of cancers often cause side effects in which pluripotential blood stem cells are damaged by the administration of a chemotherapeutic drug, irradiation of X-rays or bone marrow transplantation for the treatment of leukemia, thus decreasing the number of all hematopoietic cells. Apparently, it is markedly beneficial for thrombopenia and leukopenia patients to amplify the number of these cells by the administration of cytokine, to suppress bleeding tendency and preventing infectious diseases.

A cytokine which can amplify platelets and neutrophils simultaneously has not been found, and there is no medicine having such an effect.

Leukemia inhibiting factors, stem cell factors, macrophage colony stimulating factors, granulocyte/macrophage colony stimulating factors, erythropoietin, interleukin (IL)-3, IL-6, IL-11, megakaryocyte colony stimulating factors and the like are known as substances which amplify platelets or enhance differentiation and multiplication of megakaryocytes [Metcalf *et al., Blood,* 80, 50-56 (1990); Hunt *et al., Blood,* 80, 904-911 (1992); Examined Japanese Patent Publication No. 6-11705; Hoffman *et al., Blood Cells,* 13, 75-86 (1987); Mazur *et al., Exp. Hematol.,* 15, 1123-1133 (1987); McNiece *et al., Exp. Hematol.,* 16, 807-810 (1988); Lu *et al., Brit. J. Hematol., 70,* 149-156 (1988); Ishibashi *et al., Proc. Natl. Acad. sci. USA,* 86, 5953-5957 (1989); WO 95/21919; WO 95/18858]. It is understood that these many cytokine members amplify platelets by overlapping actions. Recently, it was revealed that a receptor ligand called c-mpl is a cytokine which has the highest activity among platelet amplifying factors and acts directly [de Sauvage *et al., Nature, 369,* 533 (1994)].

As substances which multiply granulocytes, the above-mentioned IL-3, macrophage colony stimulating factors, granulocyte/macrophage colony stimulating factors and the like are known, but G-CSF has the highest activity in terms of multiplying neutrophils selectively [Nicola *et al., J. Biol. Chem.,* 258, 9017 (1983)]. With regard to a polypeptide in which two different kinds of cytokine are fused, there are reports in Japanese Published Unexamined International Patent Application No. 500116/94, U.S. Patent 5,359,035, *Exp. Hematol.,* 21, 647-655 (1993) and *ibid.,* 18, 615 (1990) and the like.

However, nothing is known about a fusion polypeptide in which TPO is used as one of the fused cytokines.

An object of the present invention is to provide a fusion polypeptide which can produce and amplify platelets and neutrophils simultaneously. This fusion polypeptide allows the formation of megakaryocyte colonies and neutrophil colonies and the differentiation or maturation of megakaryocyte precursor and neutrophil precursor can be controlled.

DISCLOSURE OF THE INVENTION

The present invention relates to a fusion polypeptide, which comprises a polypeptide having G-CSF activity and a polypeptide having TPO activity and DNA which codes for the fusion polypeptide as defined in the claims. Also disclosed are fusion polypeptides in which a polypeptide having G-CSF activity and a polypeptide having TPO activity are fused via a spacer peptide and DNA which codes for the fusion polypeptide; and a polypeptide in which the fusion polypeptide comprising a polypeptide having G-CSF activity and a polypeptide having TPO activity is chemically modified with a polyethylene glycol derivative. Also provided are anemia-treating compositions containing the fusion polypeptide as an active ingredient.

As the polypeptide having G-CSF activity for use in the present invention, any protein may be used with the proviso that it has the requisite G-CSF activity, such as a polypeptide having the amino acid sequence shown in Table 1 [*Nature,* 319, 415 (1986)].

Also useful is a protein which has an amino acid sequence derived from the amino acid sequence shown in Table 1 by substitution, deletion or addition of one or more amino acids, and examples thereof include hG-CSF derivatives shown in Table 2 and described in Japanese Published Unexamined Patent Application No. 267299/88, Japanese Published Unexamined Patent Application No. 299/88, and Japanese Published Unexamined International Patent Application No. 500636/88.

## TABLE 1

```
X ThrProLeuGlyProAlaSerSerLeuProGlnSerPheLeuLeu
  1             5              10             15
LysCysLeuGluGlnValArgLysIleGlnGlyAspGlyAlaAlaLeu
               20             25             30
GlnGluLysLeuCysAlaThrTyrLysLeuCysHisProGluGluLeu
          35             40             45
ValLeuLeuGlyHisSerLeuGlyIleProTrpAlaProLeuSerSer
          50             55             60
CysProSerGlnAlaLeuGlnLeuAlaGlyCysLeuSerGlnLeuHis
     65             70             75
SerGlyLeuPheLeuTyrGlnGlyLeuLeuGlnAlaLeuGluGlyIle
     80             85        90             95
SerProGluLeuGlyProThrLeuAspThrLeuGlnLeuAspValAla
          100            105            110
AspPheAlaThrThrIleTrpGlnGlnMetGluGluLeuGlyMetAla
          115            120            125
ProAlaLeuGlnProThrGlnGlyAlaMetProAlaPheAlaSerAla
          130            135            140
PheGlnArgArgAlaGlyGlyValLeuValAlaSerHisLeuGlnSer
     145            150            155
PheLeuGluValSerTyrArgValLeuArgHisLeuAlaGlnPro
160            165            170            174
```

## (X represents H or Met.)

TABLE 2

| Position from N-terminal amino acid (hG-CSF in Table 1) | Substituted amino acid in hG-CSF derivatives | | | | | | | | | | | |
|---|---|---|---|---|---|---|---|---|---|---|---|---|
| | a) | b) | c) | d) | e) | f) | g) | h) | i) | j) | k) | l) |
| 1st (Thr) | * | Val | Cys | Tyr | Arg | * | Asn | Ile | Ser | * | Ala | * |

(continued)

| Position from N-terminal amino acid (hG-CSF in Table 1) | Substituted amino acid in hG-CSF derivatives | | | | | | | | | | | |
|---|---|---|---|---|---|---|---|---|---|---|---|---|
| | a) | b) | c) | d) | e) | f) | g) | h) | i) | j) | k) | l) |
| 3rd (Leu) | Glu | Ile | Ile | Ile | Thr | Thr | Glu | Thr | Thr | * | Thr | * |
| 4th (Gly) | Lys | Arg | Arg | Arg | Arg | Arg | Arg | Arg | Arg | Arg | Tyr | * |
| 5th (Pro) | Ser | Ser | Ser | Ser | Ser | Ser | Ser | Ser | Ser | * | Arg | * |
| 17th (Cys) | Ser | Ser | Ser | Ser | Ser | Ser | Ser | Ser | Ser | Ser | Ser | Ser |
| *: unsubstituted amino acid | | | | | | | | | | | | |

As the polypeptide having TPO activity for use in the present invention, any protein may be used with the proviso that it has the requisite TPO activity, such as the c-mpl ligand which is a polypeptide Having the amino acid sequence shown in Table 3 [*Nature,* 369, 533 (1994)], as well as leukemia inhibiting factors, stem cell factors, macrophage colony stimulating factors, granulocyte/macrophage colony stimulating factors, erythropoietin, interleukin (IL)-3, IL-6, IL-11, megakaryocyte colony stimulating factors and the like.

## TABLE 3

```
SerProAlaProProAlaCysAspLeuArgValLeuSerLysLeu
1           5           10              15
LeuArgAspSerHisValLeuHisSerArgLeuSerGlnCysPro
           20              25              30
GluValHisProLeuProThrProValLeuLeuProAlaValAsp
           35              40              45
PheSerLeuGlyGluTrpLysThrGlnMetGluGluThrLysAla
           50              55              60
GlnAspIleLeuGlyAlaValThrLeuLeuLeuGluGlyValMet
           65              70              75
AlaAlaArgGlyGlnLeuGlyProThrCysLeuSerSerLeuLeu
           80              85              90
GlyGlnLeuSerGlyGlnValArgLeuLeuLeuGlyAlaLeuGln
           95              100             105
SerLeuLeuGlyThrGlnLeuProProGlnGlyArgThrThrAla
           110             115             120
HisLysAspProAsnAlaIlePheLeuSerPheGlnHisLeuLeu
           125             130             135
ArgGlyLysValArgPheLeuMetLeuValGlyGlySerThrLeu
           140             145             150
CysValArgArgAlaProProThrThrAlaValProSerArgThr
           155             160             165
SerLeuValLeuThrLeuAsnGluLeuProAsnArgThrSerGly
           170             175             180
LeuLeuGluThrAsnPheThrAlaSerAlaArgThrThrGlySer
           185             190             195
GlyLeuLeuLysTrpGlnGlnGlyPheArgAlaLysIleProGly
           200             205             210
LeuLeuAsnGlnThrSerArgSerLeuAspGlnIleProGlyTyr
           215             220             225
LeuAsnArgIleHisGluLeuLeuAsnGlyThrArgGlyLeuPhe
           230             235             240
ProGlyProSerArgArgThrLeuGlyAlaProAspIleSerSer
           245             250             255
GlyThrSerAspThrGlySerLeuProProAsnLeuGlnProGly
           260             265             270
TyrSerProSerProThrHisProProThrGlyGlnTyrThrLeu
           275             280             285
PheProLeuProProThrLeuProThrProValValGlnLeuHis
           290             295             300
ProLeuLeuProAspProSerAlaProThrProThrProThrSer
           305             310             315
ProLeuLeuAsnThrSerTyrThrHisIleSerGlnAsnLeuSerGln
           320             325             330
GluGly
           332
```

The polypeptide having G-CSF activity and the other polypeptide having TPO activity, which constitute the fused polypeptide of the present invention, are not particularly limited, provided that they contain respective activity-producing portions. For example, when the c-mp1 ligand is used as the polypeptide having TPO activity, it may contain an amino acid sequence of the 153rd and 154th positions counting from the N-terminal amino acid.

Also included in the polypeptide of the present invention is a polypeptide in which a polypeptide having G-CSF activity and a polypeptide having TPO activity are fused via a spacer peptide. As the spacer peptide, any sequence may be used with the proviso that it does not spoil the G-CSF activity and TPO activity. For example, the peptide shown in Table 4 can be used as the spacer peptide.

TABLE 4

| Linker |
| --- |
| (GlyGlyGlySer)$_3$Arg |
| (SerGlyGlyGly)$_4$Arg |
| SerGlyGlyGlyArg |
| (SerGlyGlyGly)$_4$ |
| SerGlyGlyGly |
| (GlyGlyGlySer)$_3$ |
| (GlyGlyGlySer)$_2$ |

Examples of the fusion polypeptide of the present invention include a polypeptide having the amino acid sequence shown in Sequence ID No. 1, 2 or 3 and a polypeptide derived from the amino acid sequence of the fusion polypeptide by addition, deletion or substitution of one or more amino acids within such a range that the G-CSF activity and TPO activity are not spoiled, having a homology of 40% or more with the amino acid sequence of the polypeptide. The homology is preferably 60% or more, and more preferably 80% or more.

The substitution, deletion or addition of amino acids can be carried out in accordance with known methods described for example in *Nucleic Acid Research,* 10, 6487 (1982); *Proc. Natl. Acad. Sci. , USA,* 79, 6409 (1982) ; *Proc. Natl. Acad. Sci., USA,* 81, 5662 (1984); *Science,* 224, 1431 (1984); PCT WO 85/00817; *Nature,* 316, 601 (1985); *Gene,* 34, 315 (1985); *Nucleic Acid Research,* 13, 4431 (1985); and "Current Protocols in Molecular Biology", Chap. 8, Mutagenesis of Cloned DNA, John Wiley & Sons, Inc. (1989).

Also included in the fusion polypeptide of the present invention is a peptide having an amino acid sequence in which a secretion signal peptide is added to the N-terminal amino acid of the above-mentioned polypeptide; examples include a polypeptide having the amino acid sequence shown in Sequence ID No. 4, 5 or 6.

In addition, a fusion polypeptide having G-CSF activity and TPO activity, in which at least one amino group of the above-mentioned polypeptide is chemically modified with a polyalkylene glycol derivative, is also included in the fusion polypeptide of the present invention.

Examples of the polyalkylene derivative include a polyethylene glycol derivative, a polypropylene glycol derivative, a polyoxyethylene-polyoxypropylene copolymer derivative and the like. Polyethylene glycol-succinimidyl propionate is preferred.

The fusion polypeptide chemically modified with a polyethylene glycol derivative can be prepared in accordance with the method described in Japanese Examined Patent Publication No. 96558/95.

The DNA which codes for the fusion polypeptide (hereinafter referred to as "TPO-CSF") of the present invention can be obtained by polymerase chain reaction (PCR) and the like based on the known nucleotide sequences of a polypeptide having TPO activity and a polypeptide having G-CSF activity. It can also be obtained by chemical synthesis.

Examples of DNA which codes for TPO-CSF include a DNA containing a nucleotide sequence that codes for a polypeptide having the amino acid sequence shown in Sequence ID No. 1, 2 or 3 or a polypeptide derived from the amino acid sequence of the polypeptide by substitution, deletion or addition of one or more amino acids but having the G-CSF activity and TPO activity, such as a DNA which contains the nucleotide sequence shown in Sequence ID No. 4, 5 or 6.

Other examples are DNA's in which mutation such as substitution mutation, deletion mutation, insertion mutation or the like is introduced into the above-mentioned DNA within such a range that the G-CSF activity and TPO activity are not spoiled, which can be obtained, for example, by colony hybridization or plaque hybridization using a DNA containing the nucleotide sequence shown in Sequence ID No. 4, 5 or 6 as a probe.

An example is a DNA which is identified by carrying out hybridization of a membrane filter on which colony- or plaque-originated DNA is fixed, at 65°C in the presence of 0.7 to 1.0 M sodium chloride using a DNA containing the nucleotide sequence shown in Sequence ID No. 4, 5 or 6 as a probe, and subsequently washing the resulting filter at 65°C in 0.1 to 2-fold SSC solution (1-fold SSC contains 150 mM sodium chloride and 15 mM sodium citrate).

The hybridization techniques are described in "Molecular Cloning, A laboratory manual", second edition (edited by Sambrook, Fritsch and Maniatis, Cold Spring Harbor Laboratory Press, 1989).

All polypeptides encoded by the DNA defined in the foregoing are included in the TPO-CSF.

Examples of plasmids containing the TPO-CSF-encoding DNA include pBS-T153LND28, pBS-T154ND28 and pBS-T153ND28LN1. *Escherichia coli* TLN-1 as a colon bacillus containing pBS-T153LND28 and *Escherichia coli* TN-1 as a colon bacillus containing pBS-T154ND28 have been deposited on February 16, 1995, in National Institute of Bioscience and Human-Technology, Agency of Industrial Science and Technology, Higashi 1-1-3, Tsukuba-shi, Ibaraki, Japan (the postal code: 305), and have been assigned the designations as FERM BP-5001 and FERM BP-5002, respectively.

In order to express the thus obtained TPO-CSF-encoding gene (hereinafter referred to as "TPO-CSF gene") in a host, a DNA fragment containing the TPO-CSF gene is first cleaved into a TPO-CSF gene-containing DNA of an appropriate length with restriction enzymes or DNA hydrolyzing enzymes and inserted into downstream site of a promoter gene on an expression vector and then the thus DNA-inserted expression vector is introduced into a host suitable for the expression vector.

As the host, any host capable of expressing the intended gene can be used. Examples thereof include microbial strains belonging to the genera *Escherichia, Serratia, Corynebacterium, Brevibacterium, Pseudomonas, Bacillus* and the like, as well as yeast strains, animal cell hosts and the like.

Useful as the expression vector is a vector which can replicate by itself in the above-mentioned host or can be inserted into its chromosome and has a promoter at a site where transcription of the TPO-CSF gene can be made.

When a microorganism such as *Escherichia coli* or the like is used as the host, it is desirable that the TPO-CSF expression vector can replicate by itself in the microorganism and comprises a promoter, a ribosome binding sequence, the TPO-CSF gene and a transcription termination sequence. It may also contain a regulatory gene.

Examples of the expression vector include pBTrp2, pBTacl and pBTac2 (all available from Boehringer-Mannheim Co.), pKYP10 (Japanese Published Unexamined Patent Application No. 110600/83), pKYP200 [*Agric. Biol. Chem. , 48*, 669 (1984)], pLSA1 [*Agric. Biol. Chem. , 53*, 277 (1989)], pGEL1 [*Proc. Natl. Acad. Sci., USA, 82*, 4306 (1985)], pBluescript (available from STRATAGENE Co.), pTrs30 [prepared from *Escherichia coli* JM109/pTrs30 (FERM BP-5407)], pTrs32 [prepared from *Escherichia coli* JM109/pTrs32 (FERM BP-5408)], pAGE107 [Japanese Published Unexamined Patent Application No. 22979/91; Miyaji *et al., Cytotechnology, 3*, 133 (1990)], pAS3-3 (Japanese Published Unexamined Patent Application No. 227075/90) and pAMoERC3Sc CDM8 [Brian Seed *et al., Nature, 329*, 840 (1987)].

As the promoter, any one capable of exerting expression in a host such as *Escherichia coli* or the like can be used. Examples thereof include promoters originated from *Escherichia coli,* phages and the like, such as trp promoter (Ptrp), lac promoter (Plac), $P_L$ promoter, $P_R$ promoter and the like. Also useful are artificially designed and modified promoters such as a promoter prepared by connecting two Ptrp promoters in series (Ptrpx 2), tac promoter and the like.

As the ribosome binding sequence, any sequence capable of exerting expression in a host such as *Escherichia coli* or the like can be used, but it is desirable to use a plasmid in which the ribosome binding sequence and the initiation codon are arranged with an appropriate distance (for example, 6 to 18 bases).

Any gene which codes for TPO-CSF can be used as the TPO-CSF gene, but it is desirable to use the gene by substituting its bases in such a manner that the DNA sequence of the gene has codons most suitable for its expression in host microorganisms.

Although the transcription termination sequence is not always necessary for the expression of the gene, it is desirable to arrange the transcription termination sequence preferably just downstream of the structural gene.

Examples of the host include *Escherichia coli* XL1-Blue, *Escherichia coli* XL2-Blue, *Escherichia coli* DH1, *Escherichia coli* DH5 α, *Escherichia coli* MC1000, *Escherichia coli* KY3276, *Escherichia coli* W1485, *Escherichia coli* JM109, *Escherichia coli* HB101, *Escherichia coli* No. 49, *Escherichia coli* W3110, *Escherichia coli* NY49, *Bacillus subtilis, Bacillus amyloliquefacience, Brevibacterium immariophilum* ATCC 14068, *Brevibacterium saccharolyticum* ATCC 14066, *Brevibacterium flavum* ATCC 14067, *Brevibacterium lactofermentum* ATCC 13869, *Corynebacterium glutamicum* ATCC 13032, *Corynebacterium acetoacidophilum* ATCC 13870, *Microbacterium ammoniaphilum* ATCC 15354 and the like.

When a yeast strain is used as the host, YEp13 (ATCC 37115), YEp24 (ATCC 37051), YCp50 (ATCC 37419) or the like may be used as the expression vector.

Any type of promoter can-be used, provided that it can exert expression in yeast strain hosts. Examples thereof include promoters of genes of hexose kinase and the like glycolytic pathway enzymes, gal 1 promoter, gal 10 promoter, heat shock protein promoter, MF α1 promoter, CUP 1 promoter and the like.

Examples of the host include *Saccharomyces cerevisiae, Schizosaccharomyces pombe, Kluyveromyces lactis, Trichosporon pullulans, Schwanniomyces alluvius* and the like.

When animal cells are used as the host, examples of useful expression vectors include pcDNA I/Amp, pcDNA I, pcDM8 (all available from Funakoshi Co., Ltd.), pcDNA 3 (available from Invitrogen Co.), pAGE248, pAGE210 and the like.

Any promoter capable of exerting expression in the animal cell hosts can be used. For example, the promoter of human CMV IE (immediate early) gene may be used. Also, the enhancer of human CMV IE gene may be used together with the promoter.

Any gene which codes for TPO-CSF can be used as the TPO-CSF gene.

In general, only a portion of TPO-CSF expressed from the gene is secreted into the extracellular moiety, so that, in order to effect positive extracellular secretion of TPO-CSF from the host, it is desirable to prepare and use a gene having a sequence in which a nucleotide sequence coding for a signal peptide is added to the gene, in accordance with the method of Paulson *et al.* [C. Paulson *et al., J. Biol. Chem.,* 264, 17619 (1989)] and the method of Lowe *et al.* [John. B. Lowe *et al., Proc. Natl. Acad. Sci., USA,* 86, 8227 (1989); John. B. Lowe *et al., Genes Develop.,* 4, 1288 (1990)].

As the host, namalwa cells, HBT5637 (Japanese Published Unexamined Patent Application No. 299/88), COS cells, CHO cells and the like may be used.

Introduction of TPO-CSF gene-containing DNA into animal cells can be effected by any method, provided that it can introduce DNA into animal cells. For example, an electroporation method [Miyaji *et al., Cytotechnology,* 3, 133 (1990)], a calcium phosphate method (Japanese Published Unexamined Patent Application No. 227075/90), a lipofection method [Philip L. Felgner *et al., Proc. Natl. Acad. Sci., USA,* 84, 7413 (1987)] and the like may be used. Isolation and cultivation of a transformant can be effected in accordance with the method described in Japanese Published Unexamined Patent Application No. 227075/90 or Japanese Published Unexamined Patent Application No. 257891/90.

TPO-CSF can be produced by cultivating the thus obtained transformant in accordance with the usually used cultivating method.

When a transformant obtained by using *Escherichia coli,* yeast or the like microorganism as the host is cultivated, the medium may be either a natural medium or a synthetic medium, with the proviso that it contains carbon sources, nitrogen sources, inorganic salts and the like which can be assimilated by the microorganism and cultivating of the transformant can be made efficiently.

As the carbon sources, those which can be assimilated by respective microorganisms are used, which include carbohydrates such as glucose, fructose, sucrose, molasses containing them, starch, starch hydrolyzates and the like, organic acids such as acetic acid, propionic acid and the like and alcohols such as ethanol, propanol and the like.

Examples of useful nitrogen sources include ammonia, ammonium salts of various inorganic and organic acids, such as ammonium chloride, ammonium sulfate, ammonium acetate, ammonium phosphate and the like, and other nitrogen-containing compounds, as well as peptone, meat extract, yeast extract, corn steep liquor, casein hydrolyzate, soybean cake and soybean cake hydrolyzate, various fermented microbial cells and digests thereof.

Examples of useful inorganic materials include potassium dihydrogenphosphate, dipotassium hydrogenphosphate, magnesium phosphate, magnesium sulfate, sodium chloride, ferrous sulfate, manganese sulfate, copper sulfate, calcium carbonate and the like.

Cultivation is carried out under aerobic conditions by shaking, submerged-aerial stirring or the like. The temperature for the cultivation is preferably 15 to 40°C, and the period for the cultivation is generally 16 to 96 hours. The medium pH is controlled at 3.0 to 9.0 during the cultivation. Adjustment of the pH is carried out using an inorganic or organic acid, an alkaline solution, urea, calcium carbonate, ammonia and the like.

As occasion demands, antibiotics such as ampicillin, tetracycline and the like may be added to the medium during the cultivation.

When a microorganism transformed with an expression vector prepared using an inducible promoter is cultivated, an inducer may be added to the medium as occasion demands. For example, isopropyl-β-D-thiogalactopyranoside (IPTG) or the like may be added to the medium when a microorganism transformed with an expression vector prepared using lac promoter is cultivated, or indoleacetic acid (IAA) or the like when a microorganism transformed with an expression vector prepared using trp promoter is cultivated.

When a transformant obtained using animal cells as the host is cultivated, generally used RPMI 1640 medium, MEM medium (manufactured by Eagle Co. or GibcoBRL Co.), D-MEM medium (manufactured by GibcoBRL Co.) or any one of these media further supplemented with fetal bovine serum and the like may be used.

The cultivation is carried out, for example, in the presence of 5% $CO_2$. The temperature for the cultivation is preferably 35 to 37°C, and the period for the cultivation is generally 3 to 7 days.

As occasion demands, antibiotics such as kanamycin, penicillin and the like may be added to the medium during the cultivation.

Productivity can be increased using a gene amplification system in which dihydrofolate reductase gene and the like are used, in accordance with the method described in Japanese Published Unexamined Patent Application No. 227075/90.

The TPO-CSF of the present invention obtained in this manner can be purified by commonly used protein purification techniques.

For example, when the TPO-CSF is not secreted into outside moiety of the host cells, a culture broth of the transformant is subjected to centrifugation to collect cells in the culture broth, and the thus collected cells are washed and then disrupted using a sonicator, French press, Manton Gaulin homogenizer, Dynomil or the like, thereby obtaining a cell-free extract. Thereafter, the cell-free extract is subjected to centrifugation, and the TPO-CSF is purified from the resulting supernatant fluid making use of various techniques including salting out with ammonium sulfate or the like salt, anion exchange chromatography on diethylaminoethyl (DEAE)-Sepharose or the like, hydrophobic chromatography on Butylsepharose, Phenylsepharose or the like, molecular sieve-aided gel filtration and various types of electrophoresis such as isoelectric focusing and the like.

When the TPO-CSF is secreted, purified TPO-CSF can be obtained from a culture filtrate of the transformant in the same manner as the case of the above-mentioned treatment of cell-free extract supernatant.

When produced in *Escherichia coli* cells, it can be purified efficiently by the combination of the above-mentioned method with the method described in Japanese Published Unexamined Patent Application No. 267292/88.

Also, it is possible to produce the TPO-CSF of the present invention in the form of its fusion protein with another protein and to purify the product by affinity chromatography using a substance having affinity for the fused protein. For example, it is possible to produce the TPO-CSF of the present invention as its fusion protein with protein A and purify it by an immunoglobulin G-aided affinity chromatography, in accordance with the method of Lowe *et al.* [John. B. Lowe *et al., Proc. Natl. Acad. Sci., USA,* 86, 8227 (1989); John. B. Lowe *et al., Genes Develop.,* 4, 1288 (1990)].

In addition, it can also be purified by affinity chromatography using antibodies specific for a polypeptide which has G-CSF activity, such as antibodies specific for G-CSF.

The TPO-CSF of the present invention can be used as it is or as pharmaceutical compositions in various dosage forms.

The pharmaceutical compositions of the present invention are produced by mixing an effective amount of TPO-CSF as the active ingredient uniformly with pharmacologically acceptable carriers.

Preferably, these pharmaceutical compositions may be prepared in the form of unit dose packages suitable for injection.

Injections for use in injection administration can be prepared by using a carrier such as distilled water, a salt solution of sodium chloride or of a mixture of sodium chloride with other inorganic salts, a sugar solution of mannitol, lactose, dextran, glucose or the like, an amino acid solution of glycine, arginine or the like, an organic acid solution, an organic base solution or a mixture solution comprising a salt solution and a sugar solution. In that case, the composition can be made into solutions, suspensions or dispersions in the usual way using auxiliaries which include an osmotic pressure adjusting agent, a plant oil such as sesame oil or soybean oil and a surface active agent such as lecithin or a nonionic surface active agent. These solutions can be made into solid preparations by powder making, freeze drying and the like means, which are dissolved again prior to their use.

The above-mentioned pharmaceutical compositions which contain the TPO-CSF of the present invention as the active ingredient are useful for the treatment anemia or patients who become anemic as a result of treatment of diseases.

## BRIEF EXPLANATION OF THE DRAWINGS

Fig. 1 is an illustration showing construction of a plasmid containing DNA which codes for TPO-ND28 (1).

Fig. 2 is an illustration showing construction of a plasmid containing DNA which codes for TPO-ND28 (2).

Fig. 3 is an illustration showing construction of a plasmid containing DNA which codes for TPO-ND28 (3).

## BEST MODE OF CARRYING OUT THE INVENTION

### Example 1 Preparation of DNA which codes for TPO-CSF

A DNA which codes for TPO-CSF was prepared in the following manner, using a DNA which codes for a polypeptide ND28 in which the 1st position amino acid residue of the amino acid sequence of human G-CSF was substituted by alanine (Ala), and the 3rd position amino acid by threonine (Thr), the 4th position amino acid by tyrosine (Tyr), the 5th position amino acid by arginine (Arg) and the 17th position amino acid by serine (Ser) (Japanese Published Unexamined Patent Application No. 267292/88) as a DNA which codes for a polypeptide having G-CSF activity, and a DNA that codes for a polypeptide having the amino acid sequence of Table 3 (de Sauvage *et al.,* Nature, 369, 533 (1994); hereinafter referred to as "TPO") as a DNA which codes for a polypeptide having TPO activity. The fusion polypeptide of TPO and ND28 is abbreviated as TPO-ND28 hereinafter.

### 1. Preparation of TPO gene

A TPO-encoding gene (hereinafter referred to as "TPO gene") for use in the preparation of TPO-ND28 was obtained by PCR in the following manner on the basis of the nucleotide sequence reported by de Sauvage *et al.* [*Nature,* 369, 533 (1994)].

A DNA shown in Sequence ID No. 7 containing 5' end nucleotide sequence of the TPO gene (hereinafter referred to as "primer 1") and a DNA shown in Sequence ID No. 8 containing 3' end nucleotide sequence of the TPO gene (hereinafter referred to as "primer 2") were synthesized using 380A DNA synthesizer of Applied Biosystems, Inc. In order to facilitate the cloning, a restriction enzyme recognition sequence was added to the terminus of each primer.

Amplification and cloning of the TPO gene translation region sequence were carried out by reverse transcription PCR using the primers 1 and 2, human liver poly A+ mRNA (manufactured by Clontech Co., product No. CL 6510-1) mRNA and SuperScript Preamplification System for First Strand cDNA Synthesis Kit (manufactured by GibcoBRL Co.).

A 0.013 ml portion of aqueous solution containing 1,000 ng of human liver poly A+ mRNA and 500 ng of oligo (dt) 12-18 (included in the kit) was treated at 70°C for 10 minutes and then allowed to stand in ice for 1 minute.

The resulting solution was mixed with 0.002 ml of ten times-concentrated synthesis buffer, 0.001 ml of 10 mM dNTP mix, 0.002 ml of 0.1 M DTT and 0.001 ml of SuperScript II RT (200 kU/ml) (all included in the kit), and the mixture was allowed to stand at room temperature for 10 minutes and then incubated at 42°C for 50 minutes. After completion of the incubation, the mixture was heated at 90°C for 5 minutes to terminate the reverse transcription reaction.

The reaction solution was mixed with 0.001 ml of E. coli RNase H (2,000 U/ml; included in the kit) and incubated at 37°C for 20 minutes.

A 0.1 ml portion of a reaction solution containing 0.005 ml of the above reaction solution, 400 nM of the primer 1, 400 nM of the primer 2, 20 mM of Tris-HCl (pH 8.2), 10 mM of potassium chloride, 0.01 mg/ml of bovine serum albumin (hereinafter referred to as "BSA"), 2 mM of magnesium chloride, 6 mM of ammonium sulfate, 0.1% Triton X-100, 10% dimethyl sulfoxide (hereinafter referred to as "DMSO"), 0.05 mM of deoxyadenosine triphosphate (hereinafter referred to as "dATP"), 0.05 mM of deoxycytidine triphosphate (hereinafter referred to as "dCTP"), 0.05 mM of deoxyguanosine triphosphate (hereinafter referred to as "dGTP") and 0.05 mM of deoxythymidine triphosphate (hereinafter referred to as "dTTP") was mixed with 2.5 units of Pfu polymerase (manufactured by Stratagene Co.) to carry out PCR using PERKIN ELMER CETUS DNA Thermal Cycler (manufactured by Takara Shuzo Co., Ltd.) by 35 time repetition of a three step incubation at 94°C for 45 seconds, at 50°C for 1 minute and at 72°C for 2 minutes.

The resulting reaction solution was subjected to phenol/chloroform extraction and ethanol precipitation, and the thus obtained precipitate was dissolved in 0.015 ml of TE buffer [10 mM Tris-HCl (pH 8.0) and 1 mM ethylenediaminetetraacetic acid (hereinafter referred to as "EDTA")].

The thus prepared solution was mixed with restriction enzymes HindIII and KpnI to cleave the DNA amplified by PCR.

The resulting solution was subjected to an agarose gel electrophoresis, and a HindIII-KpnI treated DNA of about 1.1 kb was isolated from the agarose gel.

Using DNA Ligation Kit Ver. 1 (manufactured by Takara Shuzo Co., Ltd.), the thus isolated DNA (50 ng) was ligated with a HindIII-KpnI cleaved 2.9 kb fragment (30 ng) of a plasmid vector pBlueScript II SK(-) having a multicloning site (manufactured by Stratagene Co.) (volume of the reaction solution: 0.018 ml).

Using this reaction solution, an *Escherichia coli* strain DH5α (Library Efficiency DH5α Competent Cell, manufactured by GibcoBRL Co.) was transformed in the usual way, and the resulting transformant was spread on LB agar medium containing 50 μg/ml of ampicillin and cultivated overnight at 37°C.

Plasmids were isolated from several transformant strains grown on the medium in accordance with a known method [Birnboim *et al., Nucleic Acids Res.,* 7, 1513 (1979)].

Nucleotide sequence of the insertion fragment in each plasmid was determined using Taq DyeDeoxy Terminator Cycle Sequencing Kit (manufactured by Applied Biosystems Japan Inc., product No. 401113) and ABI373A DNA Sequencer (manufactured by Applied Biosystems Japan Inc.). In determining the nucleotide sequence, six DNA's having the nucleotide sequences of Sequence ID Nos. 9 to 13 or 14 and two primers having the nucleotide sequence shown in Sequence ID No. 15 or 16 containing a nucleotide sequence in the vector were synthesized based on the nucleotide sequence of TPO gene [de Sauvage *et al., Nature,* 369, 533 (1994)] and used as primers for the nucleotide sequence determination.

Determination of nucleotide sequence was carried out in accordance with the instructions attached to the kit and apparatus.

Of the above-mentioned plasmids, a plasmid pBS-TP0332 which coincided with the reported nucleotide sequence of the insertion fragment of TPO gene was used in the subsequent procedures.

2. Construction and expression of DNA which codes for TPO-ND28

Using the TPO-encoding DNA obtained in Example 1-1 and the ND28-encoding DNA obtained by the method described in Japanese Published Unexamined Patent Application No. 267292/88, a fusion polypeptide of TPO and ND28 (TPO on the N-terminal side and ND28 on the C-terminal side), TPO-ND-28, was prepared in the following manner.

1) Construction of DNA (Sequence ID No. 5) which codes for TPO-ND28 (1) [Sequence ID No. 2; a type constructed through a linker (Gly Gly Gly Ser Gly Gly Gly Ser Gly Gly Gly Ser Arg: sequence ID No. 17)]

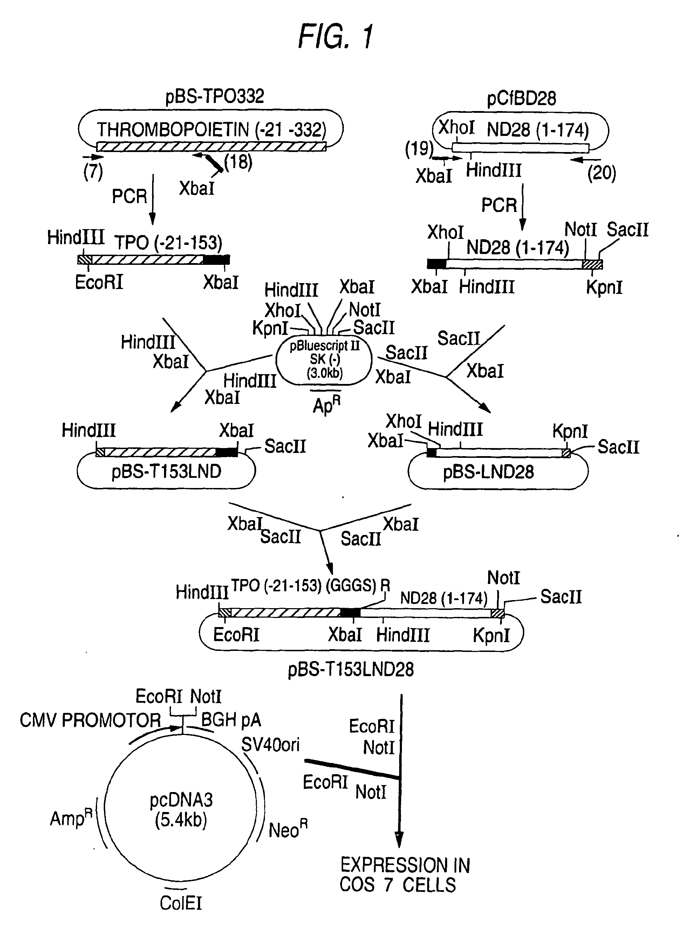

Though the mature type TPO comprises 332 amino acids, it is reported that its shortened protein consisting of its N-terminal side 153 amino acids can show the same activity of the complete length TPO [de Sauvage *et al., Nature,* 369, 533 (1994)], so that a DNA-which codes for TPO-ND28 (1) in which the 153 amino acids from the N-terminal of TPO, used as its N-terminal side, was fused with the complete length ND28 (174 amino acids) as the C-terminal side through a linker (Gly Gly Gly Ser Gly Gly Gly Ser Gly Gly Gly Ser Arg) was prepared in the following manner (cf. Fig. 1).

(i) Preparation of DNA which codes for the TPO moiety of TPO-ND28 (1)

In order to prepare a DNA which codes for the TPO moiety of TPO-ND28 (1) by means of PCR, a DNA primer having a nucleotide sequence (Sequence ID No. 18) which corresponds to the linker was synthesized as the 3' end primer (hereinafter referred to as "primer 3").

Using the thus synthesized primer 3 and the primer 1 and pBS-TP0332, PCR was carried out in the following manner.

A 0.1 ml portion of a reaction solution containing 10 ng of pBS-TP0332, 400 nM of the primer 3, 400 nM of the primer 1, 20 mM of Tris-HCl (pH 8.2), 10 mM of potassium chloride, 0.01 mg/ml of BSA, 2 mM of magnesium chloride, 6 mM of ammonium sulfate, 0.1% Triton X-100, 10% DMSO, 0.05 mM of dATP, 0.05 mM of dCTP, 0.05 mM of dGTP and 0.05 mM of dTTP was mixed with 2.5 units of Pfu polymerase to carry out PCR using PERKIN ELMER CETUS DNA Thermal Cycler (manufactured by Takara Shuzo Co., Ltd.) by 18 time repetition of a three step incubation at 94°C for 45 seconds, at 50°C for 1 minute and at 72°C for 1 minute.

The resulting reaction solution was subjected to phenol/chloroform extraction and ethanol precipitation, and the thus obtained precipitate was dissolved in 0.015 ml of TE buffer.

The thus prepared solution was mixed with restriction enzymes HindIII and XbaI to cleave the DNA amplified by PCR.

The resulting solution was subjected to an agarose gel electrophoresis, and a HindIII-XbaI treated DNA fragment of about 0.6 kb was isolated from the agarose gel.

Using DNA Ligation Kit Ver. 1 (manufactured by Takara Shuzo Co., Ltd.), the thus isolated DNA fragment (100 ng) was ligated with a HindIII-XbaI cleaved 2.9 kb fragment (50 ng) of pBlueScript II SK(-) (volume of the reaction solution: 0.018 ml).

Using this reaction solution, the *Escherichia coli* strain DH5α was transformed in the usual way, and the resulting transformant was spread on LB agar medium containing 50 μg/ml of ampicillin and cultivated overnight at 37°C.

Plasmids were isolated from several transformant strains grown on the medium in Accordance with a known method.

Nucleotide sequence of the insertion fragment in each plasmid was determined using Taq DyeDeoxy Terminator Cycle Sequencing Kit and ABI373A DNA Sequencer (manufactured by Applied Biosystems Japan Inc.). In determining the nucleotide sequence, primers having the nucleotide sequences of Sequence ID Nos. 9 to 12, 15 and 16 were used as primers for the nucleotide sequence determination.

Determination of nucleotide sequence was carried out in accordance with the instructions attached to the kit and apparatus.

Of the above-mentioned plasmids, plasmid pBS-T153LND which coincided with the reported nucleotide sequence of the insertion fragment of TPO gene was used in the subsequent procedures.

(ii) Preparation of DNA which codes for the ND28 moiety of TPO-ND28 (1)

In order to prepare a DNA which codes for the ND28 moiety of TPO-ND28 (1) by means of PCR, a primer having a nucleotide sequence (Sequence ID No. 19) which corresponds to the linker and the amino acid sequence of ND28 was synthesized as the 5' end primer (hereinafter referred to as "primer 4"), and a primer having a nucleotide sequence (Sequence ID No. 20) which corresponds to the C-terminal side amino acid sequence of ND28 was synthesized as the 3' end primer (hereinafter referred to as "primer 5").

Using the thus synthesized primers and plasmid pCfBD28 (Japanese Published Unexamined Patent Application No. 267292/88), PCR was carried out in the following manner.

A 0.1 ml portion of a reaction solution containing 10 ng of pCfBD28, 400 nM of the primer 4, 400 nM of the primer 5, 20 mM of Tris-HCl (pH 8.2), 10 mM of potassium chloride, 0.01 mg/ml of BSA, 2 mM of magnesium chloride, 6 mM of ammonium sulfate, 0.1% Triton X-100, 10% DMSO, 0.05 mM of dATP, 0.05 mM of dCTP, 0.05 mM of dGTP and 0.05 mM of dTTP was mixed with 2.5 units of Pfu polymerase to carry out PCR using PERKIN ELMER CETUS DNA Thermal Cycler (manufactured by Takara Shuzo Co., Ltd.) by 18 time repetition of a three step incubation at 94°C for 45 seconds, at 50°C for 1 minute and at 72°C for 1 minute.

The resulting reaction solution was subjected to phenol/chloroform extraction and ethanol precipitation, and the thus obtained precipitate was dissolved in 0.015 ml of TE buffer.

The thus prepared solution was mixed with restriction enzymes SacII and XbaI to cleave the DNA amplified by PCR.

The resulting solution was subjected to an agarose gel electrophoresis, and a SacII-XbaI cleaved DNA fragment of about 0.5 kb was isolated from the agarose gel.

Using DNA Ligation Kit Ver. 1 (manufactured by Takara Shuzo Co., Ltd.), the thus isolated DNA fragment (100 ng) was ligated with a SacII-XbaI cleaved 2.9 kb fragment (50 ng) of pBlueScript II SK(-) (volume of the reaction solution: 0.018 ml).

Using this reaction solution, the *Escherichia coli* strain DH5α was transformed in the usual way, and the resulting transformant was spread on LB agar medium containing 50 μg/ml of ampicillin and cultivated overnight at 37°C.

Plasmids were isolated from several transformant strains grown on the medium in accordance with a known method.

Nucleotide sequence of the insertion fragment in each plasmid was determined using Tag DyeDeoxy Terminator Cycle Sequencing Kit and ABI373A DNA Sequencer. In determining the nucleotide sequence, two DNA's having the nucleotide sequence of Sequence ID No. 21 or 22 containing a nucleotide sequence of the ND28-encoding DNA and two DNA's having the nucleotide sequence of Sequence ID No. 15 or 16 containing a sequence present in the vector were used as primers for the nucleotide sequence determination.

Determination of nucleotide sequence was carried out in accordance with the instructions attached to the kit and apparatus.

Of the above-mentioned plasmids, plasmid pBS-LND28 in which the nucleotide sequence of the insertion fragment coincided with the nucleotide sequences of the ND28 gene and primers was used in the subsequent procedures.

(iii) Preparation of DNA which codes for TPO-ND28 (1)

The DNA's respectively which code for the TPO moiety and ND28 moiety prepared in Example 1-2-1)-(i) and (ii) were fused in the following manner.

A 2,000 ng portion of pBS-T153LND was cleaved with restriction enzymes SacII and XbaI and subjected to an agarose gel electrophoresis to isolate a DNA fragment of about 3.5 kb.

Also, a 500 ng portion of pBS-LND28 was cleaved with restriction enzymes SacII and XbaI and subjected to an agarose gel electrophoresis to isolate a DNA fragment of about 0.5 kb.

Using DNA Ligation Kit Ver. 1 (manufactured by Takara Shuzo Co., Ltd.), the DNA fragment of about 3.5 kb (100 ng) was ligated with the DNA fragment of about 0.5 kb (100 ng) (volume of the reaction solution: 0.018 ml).

Using this reaction solution, the *Escherichia coli* strain DH5α was transformed in the usual way, and the resulting transformant was spread on LB agar medium containing 50 μg/ml of ampicillin and cultivated overnight at 37°C.

Plasmids were isolated from several transformant strains grown on the medium in accordance with a known method.

Structures of these plasmids were examined using restriction enzymes SacII and XbaI, and plasmid pBS-T153LND28 having a structure in which both of the DNA fragments are ligated with each other was used in the subsequent procedures.

2) Construction of DNA (Sequence ID No.4) which codes for TPO-ND28 (2) [Sequence ID No. 1; a type constructed without a linker]

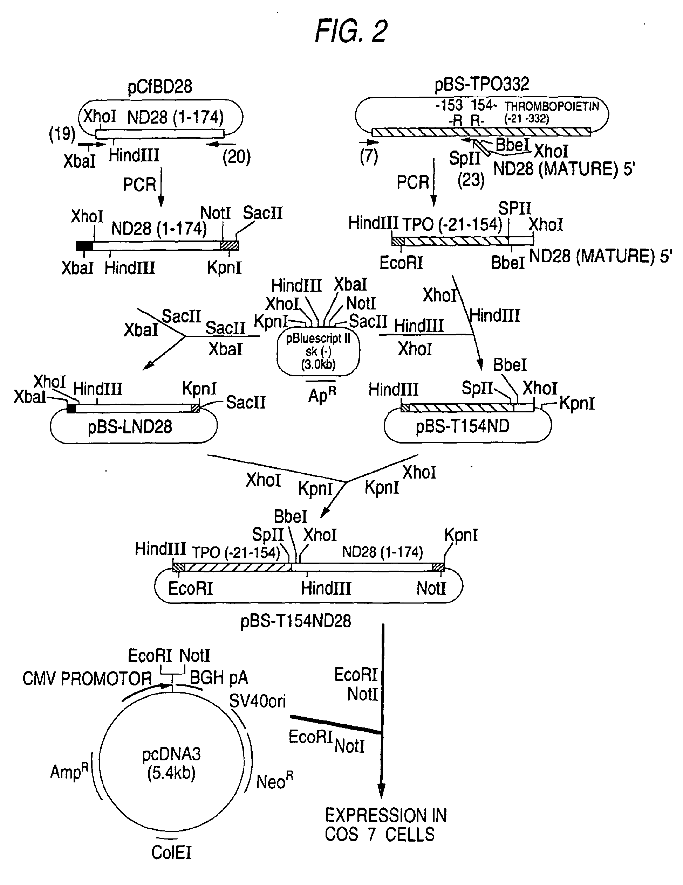

A DNA which codes for TPO-ND28 (2) in which the 154 amino acids of TPO from its N-terminal were fused with the N-terminal of D28 (174 amino acids) was prepared in the following manner (cf. Fig. 2).

(i) Preparation of DNA which codes for the TPO moiety of TPO-ND28 (2)

In order to prepare a DNA which codes for the TPO moiety of TPO-ND28 (2) by means of PCR, a primer having a nucleotide sequence shown in Sequence ID No. 23 which has a nucleotide sequence that corresponds to the amino acid sequences of TPO and ND28 was synthesized as the 3' side primer (hereinafter referred to as "primer 6").

Using the thus synthesized primer 6 and the primer 1 and pBS-TP0332, PCR was carried out in the following manner.

A 0.1 ml portion of a reaction solution containing 10 ng of pBS-TP0332, 400 nM of the primer 1, 400 nM of the primer 6, 20 mM of Tris-HCl (pH 8.2), 10 mM of potassium chloride, 0.01 mg/ml of BSA, 2 mM of magnesium chloride, 6 mM of ammonium sulfate, 0.1% Triton X-100, 10% DMSO, 0.05 mM of dATP, 0.05 mM of dCTP, 0.05 mM of dGTP and 0.05 mM of dTTP was mixed with 2.5 units of Pfu polymerase to carry out PCR using PERKIN ELMER CETUS DNA Thermal Cycler by 18 time repetition of a three step incubatiion at 94°C for 45 seconds, at 50°C for 1 minute and at 72°C for 1 minute.

The resulting reaction solution was subjected to phenol/chloroform extraction and ethanol precipitation, and the thus obtained precipitate was dissolved in 0.015 ml of TE buffer.

The thus prepared solution was mixed with restriction enzymes HindIII and XhoI to cleave the DNA amplified by PCR.

The resulting solution was subjected to an agarose gel electrophoresis, and a HindIII-XhoI cleaved DNA fragment of about 0.5 kb was isolated from the agarose gel.

Using DNA Ligation Kit Ver. 1 (manufactured by Takara Shuzo Co., Ltd.), the thus isolated DNA fragment (100 ng) was ligated with a HindIII-XhoI cleaved 2.9 kb fragment (50 ng) of pBlueScript II SK(-) (volume of the reaction solution: 0.018 ml).

Using this reaction solution, the *Escherichia coli* strain DH5α was transformed in the usual way, and the resulting transformant was spread on LB agar medium containing 50 μg/ml of ampicillin and cultivated overnight at 37°C.

Plasmids were isolated from several transformant strains grown on the medium in accordance with a known method.

Nucleotide sequence of the insertion fragment in each plasmid was determined using Taq DyeDeoxy Terminator Cycle Sequencing Kit and ABI373A DNA Sequencer (manufactured by Applied Biosystems Japan Inc.). In determining the nucleotide sequence, primers having the nucleotide sequences of Sequence ID Nos. 9 to 12, 15 and 16 were used as primers for the nucleotide sequence determination.

Determination of nucleotide sequence was carried out in accordance with the instructions attached to the kit and apparatus.

Of the above-mentioned plasmids, plasmid pBS-T154ND in which the nucleotide sequence of the insertion fragment coincided with the nucleotide sequences of the TPO gene and primers was used in the subsequent procedures.

(ii) Preparation of DNA which codes for TPO-ND28 (2)

The DNA which codes for the TPO moiety prepared in Example 1-2-2)-(i) and the DNA which codes for the ND28 moiety prepared in Example 1-2-1)-(ii) were fused in the following manner.

A 200 ng portion of pBS-T154ND was cleaved with restriction enzymes KpnI and XhoI and subjected to agarose gel electrophoresis to isolate a DNA fragment of about 3.5 kb.

Also, a 500 ng portion of pBS-LND28 was cleaved with restriction enzymes KpnI and XhoI and subjected to agarose gel electrophoresis to isolate a DNA fragment of about 0.5 kb.

Using DNA Ligation Kit Ver. 1 (manufactured by Takara Shuzo Co., Ltd.), the DNA fragment of about 3.5 kb (100 ng) was ligated with the DNA fragment of about 0.5 kb (100 ng) (volume of the reaction solution: 0.018 ml).

Using this reaction solution, the *Escherichia coli* strain DH5α was transformed in the usual way, and the resulting transformant was spread on LB agar medium containing 50 μg/ml of ampicillin and cultivated overnight at 37°C.

Plasmids were isolated from several transformant strains grown on the medium in accordance with a known method.

Structures of these plasmids were examined using restriction enzymes KpnII and XhoI, and plasmid pBS-T154ND28 having a structure in which both of the DNA fragments are ligated with each other was used in the subsequent procedures.

3) Construction of DNA (Sequence ID No. 6) which codes for TPO-ND28 (3) [Sequence ID No. 3; a type constructed through a linker (Ser Gly Gly Gly Ser Gly Gly Gly Ser Gly Gly Gly Ser Gly Gly Gly Arg; sequence ID No. 24)]

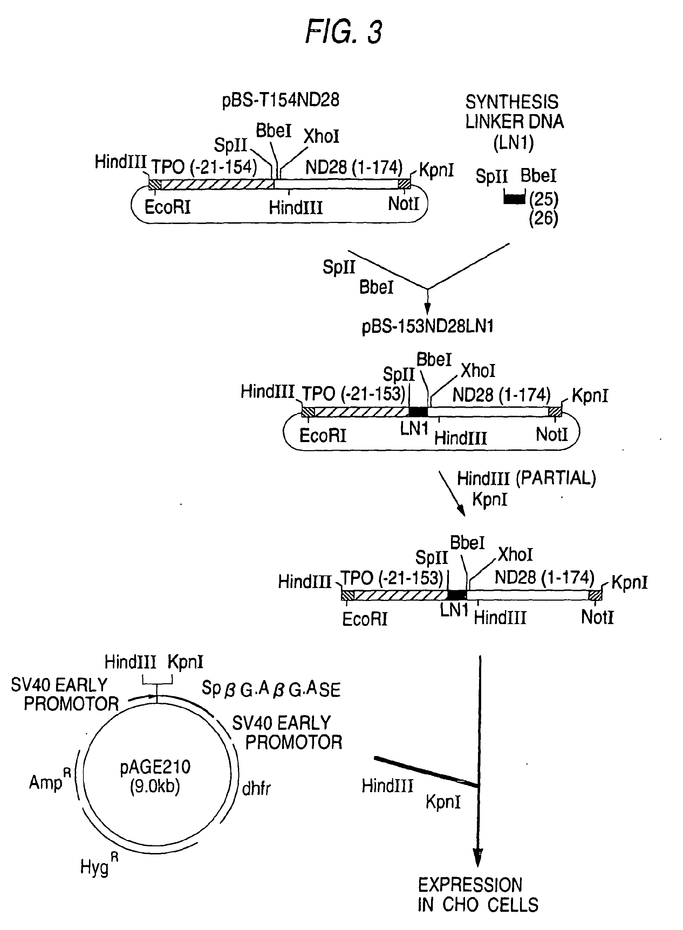

A DNA which codes for TPO-ND28 (3) in which the 153 amino acids from the N-terminal of TPO, used as its N-terminal side, was fused with the complete length ND28 (174 amino acids) as the C-terminal side through a linker (Ser Gly Gly Gly Ser Gly Gly Gly Ser Gly Gly Gly Ser Gly Gly Gly Arg) was prepared in the following manner (cf. Fig. 3).

In order to ligate the DNA which codes for the TPO moiety prepared in Example 1-2-2)-(i) with the DNA which codes for the ND28 moiety prepared in Example 1-2-1)-(ii) through a linker (Ser Gly Gly Gly Ser Gly Gly Gly Ser Gly Gly Gly Ser Gly Gly Gly Arg), two DNA's shown in Sequence ID Nos. 25 and 26 having nucleotide sequences which form SolI-BbeI complementary termini on both sides corresponding to the amino acid sequences of linkers were synthesized.

A 0.02 ml portion of a solution containing 0.01 mM of the DNA shown in Sequence ID No. 25, 5 mM of ATP, 50 mM of Tris-HCl (pH 8.0), 10 mM of magnesium chloride and 5 mM of dithiothreitol was mixed with 10 units of T4 Polynucleotide Kinase (manufactured by Takara Shuzo Co., Ltd.), and the mixture was allowed to stand at 37°C for 30 minutes and then heated at 70°C for 3 minutes to obtain treating solution (1).

The DNA shown in Sequence ID No. 26 was also treated in the same manner to obtain treating solution (2).

Treating solution (1) was mixed with treating solution (2), and the mixture was incubated at 90°C for 5 minutes and then gradually cooled to 22°C spending 3 hours to prepare double-stranded DNA.

The thus prepared double-stranded DNA was inserted into the connecting site of the TPO-coding gene and ND28-coding gene of pBS-T154ND28 obtained in Example 1-2-2)-(ii) in the following manner.

A 2,000 ng portion of pBS-T154ND28 was cleaved with restriction enzymes BbeI and SpII and subjected to an agarose gel electrophoresis to isolate a DNA fragment of about 4.0 kb.

Using DNA Ligation Kit Ver. 1 (manufactured by Takara Shuzo Co., Ltd.), the DNA fragment of about 4.0 kb (100 ng) was ligated with the above-mentioned double-stranded DNA (12.5 pmole) (volume of the reaction solution: 0.018 ml).

Using this reaction solution, the *Escherichia coli* strain DH5α was transformed in the usual way, and the resulting transformant was spread on LB agar medium containing 50 μg/ml of ampicillin and cultivated overnight at 37°C.

Plasmids were isolated from several transformant strains grown on the medium in accordance with a known method.

Nucleotide sequence of the insertion fragment in each plasmid was determined using Taq DyeDeoxy Terminator Cycle Sequencing Kit and ABI373A DNA Sequencer. In determining the nucleotide sequence, two DNA's shown in Sequence ID Nos. 12 and 22 were used as primers. Determination of nucleotide sequence was carried out in accordance with the instructions attached to the kit and apparatus.

Of these plasmids, plasmid named pBS-T153ND28LN1 in which the nucleotide sequence of the insertion fragment coincided with the nucleotide sequence of the linker DNA was used in the subsequent procedures.

Example 2 Production of TPO-CSF

The TPO-CSF was produced by effecting expression of the DNA which codes for the TPO-CSF in animal cells in the following manner.

1) Production of TPO-ND28 (1) and TPO-ND28 (2)

Plasmid pcDNA3 (manufactured by Invitrogen Co.) was cleaved with EcoRI and NotI and subjected to an agarose gel electrophoresis to isolate a DNA fragment (vector side) of about 5.4 kb.

Also, pBS-TI53LND28 and pBS-T154ND28 obtained in Example 1-2-1)-(iii) and Example 1-2-2)-(ii) were separately cleaved with EcoRI and NotI and subjected to agarose gel electrophoresis to isolate a DNA fragment (insert side) of about 1.1 kb from each plasmid.

Using DNA Ligation Kit Ver. 1, the vector side DNA fragment of about 5.4 kb (100 ng) was ligated with each of the insert side DNA fragments (100 ng) (volume of the reaction solution: 0.018 ml).

Using this reaction solution, the *Escherichia coli* strain DH5α was transformed in the usual way, and the resulting transformant was spread on LB agar medium containing 50 μg/ml of ampicillin and cultivated overnight at 37°C.

Plasmids were isolated from several transformant strains grown on the medium in accordance with a known method.

Structure of each plasmid was examined using restriction enzymes EcoRI and NotI to select plasmids containing respective inserts having a structure in which the vector side and insert side DNA fragments are ligated with each other, and plasmid pCD-153LND28 containing a TPO-ND28 (1) encoding gene and plasmid pCD-154ND28 containing a TPO-ND28 (2) encoding gene were used in the subsequent procedure.

Plasmid pCD-153LND28 or pCD-154ND28 was introduced into animal cells by electroporation [Potter *et al.*, *Proc. Natl. Acad. Sci., USA*, 81, 7161 (1984)] and its expression was effected in the following manner.

COS 7 cells were cultivated in D-MEM medium (manufactured by GibcoBRL Co., product No. 11885-50) which was further supplemented with 10% fetal bovine serum.

The COS 7 cells obtained by cultivation were suspended in K-PBS buffer (137 mM potassium chloride, 2.7 mM sodium chloride, 8.1 mM disodium hydrogenphosphate, 1.5 mM sodium dihydrogenphosphate, 4 mM magnesium chloride) to prepare a cell suspension of $8 \times 10^8$ cells/ml.

A 0.2 ml portion of the cell suspension was injected into a Pulser Cuvette (manufactured by BIO RAD LABORATORIES) having a slit width of 0.2 cm.

A 4 μg portion of pCD-153LND28 or pCD-154ND28 was added to the cuvette, thoroughly mixed with the suspension and then subjected to pulse application using an electroporation apparatus (Gene Pulser, manufactured by BIO RAD LABORATORIES) under conditions of 200 Ω, 0.3 kv/cm and 0.125 mF.

The pulse-treated solution was allowed to stand in ice for 5 minutes, suspended in 10 ml of D-MEM medium supplemented with 10% fetal bovine serum and then cultivated at 37°C for 72 hours in a $CO_2$ incubator.

The culture broth was subjected to centrifugation, and the resulting culture supernatant was filtered through a filter of 220 nm pore size to obtain a solution of TPO-ND28 (1) or TPO-ND28 (2).

2) Production of TPO-ND28 (3)

A plasmid PAGE210 was used as the vector for use in the expression of TPO-ND28 (3). The vector pAGE210 is a derivative of pAGE248 [Sasaki *et al., J. Biol. Chem.,* 269, 14730, (1994)], in which the Moloney murine leukemia virus promoter (XhoI-HindIII fragment) has been replaced by SV40 early promoter (XhoI-HindIII fragment) of pAGE103 [Mizukami *et al., J. Biochem.,* 101, 1307 (1987)].

Plasmid pAGE210 was cleaved with KpnI and HindIII and subjected to an agarose gel electrophoresis to isolate a DNA fragment (vector side) of about 9.0 kb.

Separately from this, pBS-TPO322 obtained in Example 1-1 was cleaved with KpnI and HindIII, and pBS-153ND28LN1 obtained in Example 1-2-3) was cleaved with KpnI and then partially with HindIII, and each of the resulting cleaved fragments was subjected to an agarose gel electrophoresis to isolate a DNA fragment (insert side) of about 1.1 kb from each plasmid.

Using DNA Ligation Kit Ver. 1, the vector side DNA fragment of about 9.0 kb (100 ng) was ligated with each of the insert side DNA fragments of about 1.1 kb (100 ng) (volume of the reaction solution: 0.012 ml).

Using this reaction solution, the *Escherichia coli* strain DH5α was transformed in the usual way, and the resulting transformant was spread on LB agar medium containing 50 μg/ml of ampicillin and cultivated overnight at 37°C.

Plasmids were isolated from several transformant strains grown on the medium in accordance with a known method.

Structure of each plasmid was examined using a restriction enzyme KnnI to select plasmids containing respective inserts having a structure in which the vector side and insert side DNA fragments are ligated with each other, and plasmid pAGE210-T332 containing TPO encoding gene and plasmid pAGE210-LN1 containing TPO-ND28 (3) encoding gene were used in the subsequent procedure.

Plasmid pAGE210-T332 or pAGE210-LN1 was introduced into animal cells by electroporation.

CHO cells were cultivated in MEM medium (1) (manufactured by GibcoBRL Co., product No. 19000-024) which was further supplemented with 10% fetal bovine serum.

The CHO cells obtained by cultivation were suspended in K-PBS buffer to prepare a cell suspension of $8 \times 10^6$ cells/ml.

A 0.2 ml portion of the cell suspension was injected into Pulser Cuvette having a slit width of 0.2 cm.

A 4 μg portion of pAGE210-T332 or pAGE210-LN1 was added to the cuvette, thoroughly mixed with the suspension and then subjected to pulse application using an electroporation apparatus, Gene Pulser, under conditions of 0.35 kv/cm and 0.25 mF.

The pulse-treated solution was allowed to stand in ice for 5 minutes, suspended in 10 ml of MEM medium supplemented with 10% fetal bovine serum and then cultivated at 37°C for 24 hours in a $CO_2$ incubator.

The thus cultivated cells were again cultivated for 2 weeks in MEM medium (1) supplemented with 10% fetal bovine serum and 0.3 mg/ml of hygromycin.

The resulting cells were further cultivated for 2 weeks in MEM medium (2) (manufactured by GibcoBRL Co., code No. 12000-022) supplemented with 10% fetal bovine serum and 50 nM methotrexate (hereinafter referred to as MTX).

The cultivation was repeated in the same manner by successively increasing the MTX concentration to 100 nM, 500 nM and 1,000 nM in that order, thereby obtaining strains resistant to 1,000 nM TMX.

Each of the 1,000 nM MTX resistant strains was grown in MEM medium (2) supplemented with 10% fetal bovine serum, the medium was exchanged with a serum-free medium for CHO cell use, CHO-S-SFMII (manufactured by GibcoBRL Co., code No. 12052-015), and then the strain was cultivated again for 96 to 144 hours.

By subjecting the culture broth to centrifugation, a culture supernatant containing TPO or TPO-ND28 (3) was obtained.

Example 3 Purification of TPO-ND28 (3) and TPO

A 1,000 ml portion of TPO-ND28 (3) or TPO obtained in Example 2-2) was concentrated to 50 ml using Centriprep (manufactured by Amicon Co.) to prepare a concentrated solution.

A 50 ml portion of each of the concentrated solutions was applied to XK50 column (manufactured by Pharmacia K.K.) which has been packed with 1,000 ml of Sephacryl S-200 resin (manufactured by Pharmacia K.K.) and filled with a phosphate buffer (9.4 mM sodium phosphate (pH 7.2), 137 mM NaCl, 2.7 mM KCl).

Elution of TPO-ND28 (3) or TPO was effected by passing the phosphate buffer through the column at a flow rate of 3 ml/minute.

The eluates were pooled for every 12.5 minutes, and the resulting fractions were checked for their TPO and G-CSF activities by an MTT assay method which will be described later, thereby obtaining purified TPO-ND28 (3) or TPO.

Example 4 Modification of TPO-ND28 (3) with polyethylene glycol

To ice-cooled water was added 20 kd PEG-succinimidyl propionate (manufactured by Shearwater Polymers Co.) to a final concentration of 400 mg/ml.

A 50 $\mu$l portion of the thus prepared aqueous solution was mixed with 200 $\mu$l of the TPO-ND28 (3) solution obtained in Example 3 and 150 $\mu$l of distilled water. The mixture was allowed to stand for 12 hours at 4°C, thereby effecting modification of TPO-ND28 (3) by polyethylene glycol.

The TPO-ND28 (3) thus modified with polyethylene glycol (hereinafter referred to as PEG-TPO-ND28 (3)) was applied to a column of Super Rose 610/30 (manufactured by Pharmacia K.K.) which has been filled in advance with a phosphate buffer (9.4 mM sodium phosphate (pH 7.2), 137 mM NaCl, 2.7 mM RCl).

Elution was effected by passing the phosphate buffer through the column at a flow rate of 0.5 ml/minute.

The eluates were pooled for every 1 minute, and the resulting fractions were checked for their G-CSF and TPO activities by MTT assay method which will be described later.

The results are shown in Table 5.

The G-CSF and TPO activities originated from unmodified TPO-ND28 (3) were detected 34 to 40 minutes after commencement of the elution, and the G-CSF and TPO activities originated from PEG-TPO-ND28 (3) were detected after 16 to 28 minutes of the elution.

These results confirmed that polyethylene glycol-modified TPO-CSF having both G-CSF and TPO activities can be obtained.

TABLE 5

| Elution time (minutes) | 0 | 10 | 14 | 16 | 18 | 20 | 22 | 24 | 26 | 28 | 30 | 32 | 34 | 36 | 38 | 40 |
|---|---|---|---|---|---|---|---|---|---|---|---|---|---|---|---|---|
| G-CSF activity | - | - | - | + | + | + | + | + | + | + | - | - | + | + | + | + |
| TPO activity | - | - | - | + | + | + | + | + | + | + | - | - | + | + | + | + |

-: no activity; +: activity

Test Example 1 Measurement of TPO-ND28 molecular weight

Using the TPO-ND28 (1) solution obtained in Example 2-1), its molecular weight was measured by a gel filtration chromatography in the following manner.

A 0.2 ml portion of the TPO-ND28 (1) solution was applied to a column of Super Rose 610/_30 (manufactured by Pharmacia K.K.) which has been equilibrated in advance with a phosphate buffer (9.4 mM sodium phosphate (pH 7.2), 137 mM NaCl, 2.7 mM KCl), and elution of TPO-ND28 (1) was effected by passing the phosphate buffer through the column at a flow rate of 0.5 ml/minute.

The eluates were pooled for every 0.5 minute, and the resulting fractions were checked for their TPO and G-CSF activities by an MTT assay method which will be described later.

Table 6 shows elution time from Super Rose and measured values of TPO and G-CSF activities.

The TPO and G-CSF activities reached the maximum after 33.5 minutes of the elution.

Separately from this, thyroglobulin (molecular weight: 670,000), aldolase (molecular weight: 160,000), bovine serum albumin (molecular weight: 69,000) and G-CSF (molecular weight: 20,000) were used as the standard molecular weight proteins and passed through Super Rose to obtain relationship between elution time and molecular weight.

Molecular weight of TPO-ND28 (1) deduced from the 33.5 minutes of elution time was about 40,000.

TABLE 6

| Elution time | 0 | 20 | 30 | 32 | 33 | 33.5 | 34 | 35 | 37 | 42 |
|---|---|---|---|---|---|---|---|---|---|---|
| TPO activity ($A_{540}$) | 0.08 | 0.07 | 0.08 | 0.19 | 0.30 | 0.32 | 0.29 | 0.18 | 0.09 | 0.08 |
| G-CSF activity ($A_{540}$) | 0.00 | 0.00 | 0.03 | 0.14 | 0.29 | 0.32 | 0.30 | 0.22 | 0.05 | 0.00 |

**EP 0 783 003 B1**

<u>Test Example 2 Biological activity of TPO-CSF</u>

Basic construction for the measurement of the cell growth-stimulating activity of a solution to be tested (TPO-ND28 solution) upon cells to be tested is as follows.

Each solution to be tested (TPO-ND28 solution), TPO standard solution and ND28 standard solution is made into 10-fold serial dilutions, and a 0.01 ml portion of each of the dilutions is added to each well of a microtiter plate.

Actively growing cells to be tested are collected from a culture broth by centrifugation, washed and then re-suspended in a medium for testing use to a most suitable cell density for each testing.

The thus prepared cell suspension is dispensed in 0.09 ml portions into wells of the above-mentioned microtiter plate which has been prepared by dispensing dilutions of the solution to be tested, TPO standard solution or ND28 standard solution in 0.01 ml portions.

The microtiter plate is incubated at 37°C in a completely moist 5% $CO_2$ incubator and then used in the following testing.

A 0.01 ml portion of 0.5 mg/ml solution of MTT [3-(4,5-dimethyl-2-thiazolyl)-2,5-diphenyl tetrazolium bromide] is added to each well, incubated for 4 hours, mixed with 0.15 ml of 0.1 N hydrochloric acid/isopropyl alcohol solution and then stirred to extract pigment from the cells, subsequently judging growth of the cells by measuring the amount of the pigment by its absorbance at 540 nm.

This method for the measurement of cell growth-stimulating activity is hereinafter called the MTT assay.

<u>(1) Measurement of cell growth-stimulating activity upon Ba/F3 cells</u>

The Ba/F3 cells which grow depending on the presence of mouse IL-3 were cultivated in Iscove's modified Dulbecco medium (hereinafter referred to as "IMDM") which has been supplemented with 10% heat-inactivated fetal calf serum (hereinafter referred to as "FCS") and mouse IL-3 (culture supernatant of WEHI-3B).

Using the thus cultivated Ba/F3 cells, the cell growth-stimulating activity was measured by the MTT assay using the just described medium but in the absence of mouse IL-3.

The MTT assay was carried out with an inoculation density of 10,000 cells per well and by incubating the plate in 5% $CO_2$ for 48 hours.

Results of the MTT assay showed that each of TPO, ND28 and TPO-ND28 (1), (2) and (3) had no Ba/F3 cell growth-stimulating activity.

<u>(2) Measurement of cell growth-stimulating activity upon Ba/F3-cmp1</u>

The Ba/F3-cmp1 cells which grow depending on the presence of mouse IL-3 or TPO were cultivated in IMDM which has been supplemented with 10% heat-inactivated FCS, 0.5 mg/ml of G418 and mouse IL-3 (culture supernatant of WEHI-3B).

Using the thus cultivated Ba/F3-cmpl cells, the cell growth-stimulating activity was measured by MTT assay using the just described medium but in the absence of mouse IL-3.

The MTT assay was carried out with an inoculation density of 10,000 cells per well and by incubating the plate in 5% $CO_2$ for 48 hours.

Results of the MTT assay showed that each of TPO and TPO-ND28 (1), (2) and (3) had Ba/F3-cmp cell growth-stimulating activity.

<u>(3) Measurement of cell growth-stimulating activity upon NFS-60 cells</u>

The NFS-60 cells which grow depending on the presence of human G-CSF or mouse IL-3 were cultivated in RPMI medium which has been supplemented with 10% heat-inactivated FCS, 2 mM glutamine, P/S (100 U/ml of penicillin, 100 mg/ml of streptomycin) and 1.0 ng/ml of recombinant type human G-CSF.

Using the thus cultivated NFS-60 cells, the cell growth-stimulating activity was measured by the MTT assay using the just described medium but in the absence of G-CSF.

The MTT assay was carried out with an inoculation density of 10,000 cells per well and by incubating the plate in 5% $CO_2$ for 48 hours.

Results of the MTT assay showed that each of ND28 and TPO-ND28 (1), (2) and (3) had NFS-60 cell growth-stimulating activity.

<u>Test Example 3 Effect of TPO-ND28 on mouse myeloid cells</u>

A BALB/c mouse of 8 weeks of age was sacrificed to excise the femur and tibia system whose both ends were subsequently cut with scissors. The needle of a syringe filled with RPMI solution containing 10% FCS was inserted into the section of femur and tibis to blow off myeloid cells into a small test tube, and the cells were allowed to stand for 5 minutes.

Using a Pasteur pipette, the supernatant fluid in the test tube was drawn up taking care not to contaminate it with the precipitate, and the supernatant fluid was overlaid on Nycoprep 1.077 Animal (manufactured by NYCOMED Co., product No. 1002380) and subjected to 15 minutes of centrifugation at 600 g to isolate mouse mono nuclear cells (hereinafter referred to as "MNC").

The MNC were made into a suspension of $5 \times 10^5$ cells/ml with a solution containing a solution to be tested, 10% FCS, 1% BSA and 0.6 mg/ml of transferrin (manufactured by Boehringer Manheim Co.) and cultivated for 5 days in a $CO_2$ incubator (BNA-120D, manufactured by TABAI Co.) under conditions of 37°C, 5% $CO_2$ and 95% or more of humidity.

As the solution to be tested, a solution of TPO, ND28 or TPO-ND28 having a final concentration of 1.0, 10 or 100 ng/ml or a solution in which the same volume of TPO and ND-28 solutions having the above-mentioned concentration were mixed (TPO/ND28) was used. The TPO and ND28 obtained in Example 3 were used.

After completion of the cultivation, conditions of the differentiation of MNC were examined by measuring the amount of CD61 expressed which is an index of differentiation into megakaryocyte system [*J. Med.,* 311, 1084 (1984)] and the amount of Gr-1 expressed which is an index of differentiation into the granulocyte system [*J. Immunol.,* 144, 22 (1991)].

After staining with anti mouse CD61-FITC monoclonal antibody (manufactured by PHARMINGEN Co., product No. 01864D) and anti mouse Gr-1-PE monoclonal antibody (manufactured by PHARMINGEN Co., product No. 01215A), expressed amounts of CD61 and Gr-1 were measured using an ELITE flow cytometer (manufactured by Coulter Co.).

The results are shown in Table 7.

TABLE 7

| Solution to be tested | Concentration | Expressed cells (%) | |
|---|---|---|---|
| | (ng/ml) | Gr-1 | CD61 |
| no addition | | 1.0 | 1.0 |
| ND28 | 1.0 | 49.1 | 7.6 |
| | 10.0 | 40.7 | 4.9 |
| | 100.0 | 44.5 | 4.6 |
| TPO | 1.0 | 36.7 | 8.7 |
| | 10.0 | 37.7 | 17.8 |
| | 100.0 | 37.1 | 21.9 |
| TPO/ND28 | 1.0 | 50.7 | 10.3 |
| | 10.0 | 40.6 | 10.4 |
| | 100.0 | 49.2 | 5.7 |
| TPO-ND28 | 1.0 | 50.5 | 22.1 |
| | 10.0 | 49.8 | 26.6 |
| | 100.0 | 41.0 | 18.8 |

When the solution to be tested prepared by mixing the same amount of TPO and ND28 (TPO/ND28) was added, Gr-1 expressed cells were generated in a level similar to the case of the addition of the solution to be tested containing ND28 alone, thus showing differentiation of MNC into the granulocyte system, but frequency of the generation of CD61 expressed cells was lower than the case of the addition of the solution to be tested containing TPO alone, thus showing decreased differentiation into the megakaryocyte system. These results suggest that, when the same amount of TPO and ND28 are present, MNC reacts mostly with ND28 and differentiates into the granulocyte system.

However, when the fusion polypeptide of TPO and ND28, namely TPO-ND28, was added as the solution to be tested, frequency of the generation of CD61 expressed cells was similar to or higher than the case of the addition of the solution to be tested containing TPO alone and two times or more higher than the case of the addition of TPO/ND28. What is more, the frequency of the generation of Gr-1 expressed cells was also similar to the case of the addition of the solution to be tested containing ND28 alone.

Test Example 4 Platelet and leukocyte production-enhancing function in mice

A 10μg/ml solution of TPO or a 10 μg/ml solution of TPO-ND28 (3) obtained in Example 3 was administered by subcutaneous injection to BALB/c mice (males, 7 weeks of age) with a dose of 0.2 ml per 20 g body weight of each mouse, once a day continuously for 4 days starting on the first day of the test (treated groups, 4 animals per one group). A blood sample was collected from the ophthalmic vein of each animal on the fifth day of the test to count the number of platelets and leukocytes by a microcell counter (Sysmex F800, manufactured by Toa Iyo Denshi Co.).

After introducing the plasmid pAGE210 used for the expression of TPO or TPO-ND28 (3) gene into CHO cells in accordance with the method described in Example 2-2), the cells were cultivated, the resulting culture supernatant was treated by the same TPO-ND28 (3) purification procedure described in Example 3, and an elution fraction corresponding to the elution fraction of TPO-ND28 (3) was used as a blank solution to count the number of platelets and leukocytes by the above-mentioned method.

In order to compare and examine effects of TPO and TPO-ND28 (3), the increasing ratio (%) of the number of platelets and leukocytes in the group in which each of these substances were administered to that in the blank solution-administered group was calculated based on the following formula:

```
[platelet or leukocyte counts in mice of TPO- or TPO-ND28 (3)-
administered group]/[platelet or leukocyte count in mice of
blank solution-administered group] x 100
```

The results are shown in Table 8.

TABLE 8

| Test substance | Increasing ratio of platelets | Increasing ratio of leukocytes |
|---|---|---|
| | (%) | (%) |
| TPO | 219 | 106 |
| TPO-ND28 | 170 | 160 |

INDUSTRIAL APPLICABILITY

A fusion polypeptide comprising a polypeptide having both G-CSF activity and a polypeptide having TPO activity is provided by the present invention. The fusion polypeptide of the present invention can form and amplify platelets and leukocytes simultaneously and can control formation of megakaryocyte colonies and neutrophil colonies and differentiation or maturation of megakaryocyte precursors and neutrophil precursors.

**Claims**

1. A fusion polypeptide which comprises an amino acid sequence selected from the amino acid sequences shown in SEQ ID NOs.: 1, 2 and 3.

2. A fusion polypeptide which comprises an amino acid sequence selected from the amino acid sequences as shown in SEQ ID NOs.: 1, 2 and 3, additionally containing in its granulocyte colony stimulating factor portion an amino acid substitution pattern a), b), c), d), e), f), g), h), i), j), or l) as defined in the following table

| Position from H-terminal amino acid (hG-CSF sequence below) | Substituted amine acid in hG-CSF derivatives | | | | | | | | | | | |
|---|---|---|---|---|---|---|---|---|---|---|---|---|
| | a) | b) | c) | d) | e) | f) | g) | h) | i) | j) | k) | l) |
| 1st (Thr) | * | Val | Cys | Tyr | Arg | * | Asn | Ile | Ser | * | Ala | * |
| 3rd (Leu) | Glu | Ile | Ile | Ile | Thr | Thr | Glu | Thr | Thr | * | Thr | * |
| 4th (Gly) | Lys | Arg | Arg | Arg | Arg | Arg | Arg | Arg | Arg | Arg | Tyr | * |
| 5th (Pro) | Ser | Ser | Ser | Ser | Ser | Ser | Ser | Ser | Ser | * | Arg | * |
| 17th (Cys) | Ser | Ser | Ser | Ser | Ser | Ser | Ser | Ser | Ser | Ser | Ser | Ser |
| *: unsubstituted amino acid | | | | | | | | | | | | |

or wherein the granulocyte colony stimulating factor portion is replaced by one of the following amino acid sequences

```
X ThrProLeuGlyProAlaSerSerLeuProGlnSerPheLeuLeu
1           5              10              15
LysCysLeuGluGlnValArgLysIleGlnGlyAspGlyAlaAlaLeu
        20              25              30
GlnGluLysLeuCysAlaThrTyrLysLeuCysHisProGluGluLeu
        35              40              45
ValLeuLeuGlyHisSerLeuGlyIleProTrpAlaProLeuSerSer
        50              55              60
CysProSerGlnAlaLeuGlnLeuAlaGlyCysLeuSerGlnLeuHis
        65              70              75
SerGlyLeuPheLeuTyrGlnGlyLeuLeuGlnAlaLeuGluGlyIle
        80              85              90              95
SerProGluLeuGlyProThrLeuAspThrLeuGlnLeuAspValAla
        100             105             110
AspPheAlaThrThrIleTrpGlnGlnMetGluGluLeuGlyMetAla
        115             120             125
ProAlaLeuGlnProThrGlnGlyAlaMetProAlaPheAlaSerAla
        130             135             140
PheGlnArgArgAlaGlyGlyValLeuValAlaSerHisLeuGlnSer
        145             150             155
PheLeuGluValSerTyrArgValLeuArgHisLeuAlaGlnPro
160             165             170             174

        (X represents H or Met.)
```

3. The fusion polypeptide according to claim 1 or 2, wherein the polypeptide is a polypeptide in which at least one amino group of the polypeptide is chemically modified with a polyalkylene glycol derivative.

4. The fusion polypeptide according to claim 3, wherein the polyalkylene glycol derivative is a polyethylene glycol derivative, a polypropylene glycol derivative or a polyoxyethylene-polyoxypropylene copolymer derivative.

**5.** A DNA which codes for the fusion polypeptide according to claim 1 or 2.

**6.** The DNA according to claim 5, wherein the DNA is a DNA which contains a sequence selected from the DNA sequences shown in SEQ ID NOs.: 4, 5 and 6.

**7.** An pharmaceutical, preferably anemia-treating, composition comprising the fusion polypeptide according to any one of claims 1 to 4 as an active ingredient.

**8.** Use of the fusion polypeptide according to any one of claims 1 to 4 for the manufacture of a medicament for treating anemia.

**Patentansprüche**

**1.** Fusionspolypeptid welches eine Aminosäuresequenz ausgewählt unter den Aminosäuresequenzen gemäß SEQ ID NO:1, 2 und 3 umfasst.

**2.** Fusionspolypeptid welches eine Aminosäuresequenz ausgewählt unter den Aminosäuresequenzen gemäß SEQ ID NO:1, 2 und 3 umfasst und zusätzlich in seinem Granulocyten-Kolonie-stimulierender Faktor-Abschnitt ein Aminosäuresubstitutionsmuster a), b), c), d), e), f), g), h), i), j), k) oder l) gemäß der Definition in folgender Tabelle aufweist

| Position zur N-terminalen Aminosäure (HG-CSF Sequenz unten) | Substituierte Aminosäure in hG-CSF-Derivaten | | | | | | | | | | | |
|---|---|---|---|---|---|---|---|---|---|---|---|---|
| | a) | b) | c) | d) | e) | f) | g) | h) | i) | j) | k) | l) |
| 1, (Thr) | * | Val | Cys | Tyr | Arg | * | Asn | Ile | Ser | * | Ala | * |
| 3. (Leu) | Glu | Ile | Ile | Ile | Thr | Thr | Glu | Thr | Thr | * | Thr | * |
| 4. (Gly) | Lys | Arg | Arg | Arg | Arg | Arg | Arg | Arg | Arg | Arg | Tyr | * |
| 5. (Pro) | Ser | Ser | Ser | Ser | Ser | Ser | Ser | Ser | Ser | * | Arg | * |
| 17. (Cys) | Ser | Ser | Ser | Ser | Ser | Ser | Ser | Ser | Ser | Ser | Ser | Ser |
| *: unsubstituierte Aminosäure | | | | | | | | | | | | |

oder wobei der Granulocyten-Kolonie-stimulierende Faktor-Abschnitt ersetzt ist durch eine der folgenden Aminosäuresequenzen

```
X ThrProLeuGlyProAlaSerSerLeuProGlnSerPheLeuLeu
  1           5           10          15
LysCysLeuGluGlnValArgLysIleGlnGlyAspGlyAlaAlaLeu
            20          25          30
GlnGluLysLeuCysAlaThrTyrLysLeuCysHisProGluGluLeu
            35          40          45
ValLeuLeuGlyHisSerLeuGlyIleProTrpAlaProLeuSerSer
            50          55          60
CysProSerGlnAlaLeuGlnLeuAlaGlyCysLeuSerGlnLeuHis
            65          70          75
SerGlyLeuPheLeuTyrGlnGlyLeuLeuGlnAlaLeuGluGlyIle
            80          85          90          95
SerProGluLeuGlyProThrLeuAspThrLeuGlnLeuAspValAla
            100         105         110
AspPheAlaThrThrIleTrpGlnGlnMetGluGluLeuGlyMetAla
            115         120         125
ProAlaLeuGlnProThrGlnGlyAlaMetProAlaPheAlaSerAla
            130         135         140
PheGlnArgArgAlaGlyGlyValLeuValAlaSerHisLeuGlnSer
            145         150         155
PheLeuGluValSerTyrArgValLeuArgHisLeuAlaGlnPro
            160         165         170         174
```

(X steht für H oder Met.)

3. Fusionspolypeptid nach Anspruch 1 oder 2, wobei das Polypeptid ein Polypeptid ist, in welchem wenigstens eine Aminogruppe des Polypeptides mit einem Polyalkylenglycolderivat chemisch modifiziert ist.

4. Fusionspolypeptid nach Anspruch 3, wobei das Polyalkylenglycolderivat ein Polyethylenglycolderivat, ein Polypropylenglycolderivat oder ein Polyoxyethylen-Polyoxypropylencopolymer-Derivat ist.

5. DNA, welche für das Fusionspolypeptid nach Anspruch 1 oder 2 kodiert.

6. DNA nach Anspruch 5, wobei die DNA eine DNA ist, welche eine Sequenz, ausgewählt unter DNA-Sequenzen gemäß SEQ ID NO:4, 5 und 6 enthält.

7. Pharmazeutisches Mittel, vorzugsweise zur Behandlung von Anämie, umfassend ein Fusionspolypeptid nach einem der Ansprüche 1 bis 4 als aktiven Bestandteil.

8. Verwendung des Fusionspolypeptids nach einem der Ansprüche 1 bis 4 zur Herstellung eines Medikaments zur Behandlung von Anämie.

**Revendications**

1. Polypeptide de fusion qui comprend une séquence d'acides aminés choisie parmi les séquences d'acides aminés présentées dans SEQ ID N° 1, 2 et 3.

2. Polypeptide de fusion qui comprend une séquence d'acides aminés choisie parmi les séquences d'acides aminés présentées dans SEQ ID N° 1, 2 et 3, contenant en outre dans sa partie de facteur de stimulation des colonies de granulocytes un motif de substitution d'acides aminés a), b), c), d), e), f), g), h), i), j), k) ou l) comme défini dans le tableau ci-dessous

| Position à partir de l'acide aminé N-terminal (séquence de hG-CSF ci-dessous) | Acide aminé substitué dans les dérivés d'hG-CSF | | | | | | | | | | | |
|---|---|---|---|---|---|---|---|---|---|---|---|---|
| | a) | b) | c) | d) | e) | f) | g) | h) | i) | j) | k) | l) |
| 1ère (Thr) | * | Val | Cys | Tyr | Arg | * | Asn | Iln | Ser | * | Aln | * |
| 3e (Leu) | Glu | Ile | Ile | Ile | Thr | Thr | Glu | Thr | Thr | * | Thr | * |
| 4e (Gly) | Lys | Arg | Arg | Arg | Arg | Arg | Arg | Arg | Arg | Arg | Tyr | * |
| 5e (Pro) | Ser | Ser | Ser | Ser | Ser | Ser | Ser | Ser | Ser | * | Arg | * |
| 17e (Cys) | Ser | Ser | Ser | Ser | Ser | Ser | Ser | Ser | Ser | Ser | Ser | Ser |
| • : acide aminé non substitué | | | | | | | | | | | | |

ou dans lequel la partie de facteur de stimulation de colonies est remplacée par l'une des séquences d'acides aminés suivantes

```
X ThrProLeuGlyProAlaSerSerLeuProGlnSerPheLeuLeu
1              5              10             15
LysCysLeuGluGlnValArgLysIleGlnGlyAspGlyAlaAlaLeu
             20             25             30
GlnGluLysLeuCysAlaThrTyrLysLeuCysHisProGluGluLeu
             35             40             45
ValLeuLeuGlyHisSerLeuGlyIleProTrpAlaProLeuSerSer
             50             55             60
CysProSerGlnAlaLeuGlnLeuAlaGlyCysLeuSerGlnLeuHis
             65             70             75
SerGlyLeuPheLeuTyrGlnGlyLeuLeuGlnAlaLeuGluGlyIle
             80             85             90             95
SerProGluLeuGlyProThrLeuAspThrLeuGlnLeuAspValAla
             100            105            110
AspPheAlaThrThrIleTrpGlnGlnMetGluGluLeuGlyMetAla
             115            120            125
ProAlaLeuGlnProThrGlnGlyAlaMetProAlaPheAlaSerAla
             130            135            140
PheGlnArgArgAlaGlyGlyValLeuValAlaSerHisLeuGlnSer
             145            150            155
PheLeuGluValSerTyrArgValLeuArgHisLeuAlaGlnPro
160            165            170            174
```

(X représente H ou Met).

3. Polypeptide de fusion selon la revendication 1 ou 2, dans lequel le polypeptide est un polypeptide dans lequel au moins un groupe amino du polypeptide est modifié chimiquement avec un dérivé de polyalkylène glycol.

4. Polypeptide de fusion selon la revendication 3, dans lequel le dérivé de polyalkylène glycol est un dérivé de polyéthylène glycol, un dérivé de polypropylène glycol ou un dérivé du copolymère de polyoxyéthylène - polyoxypropylène.

5. ADN qui code le polypeptide de fusion selon la revendication 1 ou la revendication 2.

6. ADN selon la revendication 5, dans lequel l'ADN est un ADN qui contient une séquence choisie parmi les séquences d'ADN présentées dans SEQ ID N° 4, 5 et 6.

7. Composition pharmaceutique, de préférence pour le traitement de l'anémie, comprenant le polypeptide de fusion selon l'une quelconque des revendications 1 à 4 comme ingrédient actif.

8. Utilisation du polypeptide de fusion selon l'une quelconque des revendications 1 à 4 pour la fabrication d'un médicament pour le traitement de l'anémie.

# FIG. 1

## FIG. 2

## FIG. 3

pBS-T154ND28

SYNTHESIS
LINKER DNA
(LN1)

BbeI
SpII XhoI
HindIII TPO (-21-154) ND28 (1-174) KpnI

SpII BbeI
(25)
(26)

EcoRI HindIII NotI

SpII
BbeI

pBS-153ND28LN1

BbeI
SpII XhoI
HindIII TPO (-21-153) ND28 (1-174) KpnI

EcoRI LN1 HindIII NotI

HindIII (PARTIAL)
KpnI

BbeI
SpII XhoI
HindIII TPO (-21-153) ND28 (1-174) KpnI

EcoRI LN1 HindIII NotI

HindIII KpnI

SV40 EARLY
PROMOTOR Spβ G.Aβ G.ASE

SV40 EARLY
PROMOTOR

Amp^R pAGE210
(9.0kb) dhfr

HindIII
KpnI

Hyg^R

EXPRESSION
IN CHO CELLS

SEQUENCE LISTING

(1) GENERAL INFORMATION:

(i) APPLICANT: Kyowa Hakko Kogyo Co., Ltd

(ii) TITLE OF INVENTION: Novel Polypeptides

(iii) NUMBER OF SEQUENCES: 26

(iv) CORRESPONDENCE ADDRESS:

(A) ADDRESSEE: Kyowa Hakko Kogyo Co., Ltd.
(B) STREET: 6-1, Ohtemachi 1-chome, Chiyoda-ku
(C) CITY: Tokyo
(E) COUNTRY: Japan

(v) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:

(A) APPLICATION NUMBER: 96 912 262.1
(B) FILING DATE: 26.04.1996

(viii) ATTORNEY/AGENT INFORMATION:

(A) NAME: Kinzebach, Werner, Dr.
(C) REFERENCE/DOCKET NUMBER: M/37549

(ix) TELECOMMUNICATION INFORMATION:

(A) TELEPHONE: (089) 998397-0
(B) TELEFAX: (089) 987304

(2) INFORMATION FOR SEQ ID NO:1:

(i) SEQUENCE CHARACTERISTICS:

(A) LENGTH: 328 amino acids
(B) TYPE: amino acid
(C) STRANDEDNESS: single
(D) TOPOLOGY: linear

(ii) MOLECULE TYPE: peptide

(vi) ORIGINAL SOURCE

(A) ORGANISM: human (Homo sapiens)

(ix) FEATURE

(A) NAME/KEY:
(B) LOCATION: 1..154

(A) NAME/KEY:
(B) LOCATION: 154..328

(xi) SEQUENCE DESCRIPTION: SEQ ID NO:1:

```
Ser Pro Ala Pro Pro Ala Cys Asp Leu Arg Val Leu Ser Lys Leu Leu
 1               5               10              15

Arg Asp Ser His Val Leu His Ser Arg Leu Ser Gln Cys Pro Glu Val
             20              25              30

His Pro Leu Pro Thr Pro Val Leu Leu Pro Ala Val Asp Phe Ser Leu
         35              40              45

Gly Glu Trp Lys Thr Gln Met Glu Glu Thr Lys Ala Gln Asp Ile Leu
     50              55              60

Gly Ala Val Thr Leu Leu Leu Glu Gly Val Met Ala Ala Arg Gly Gln
 65              70              75              80

Leu Gly Pro Thr Cys Leu Ser Ser Leu Leu Gly Gln Leu Ser Gly Gln
             85              90              95
```

Val Arg Leu Leu Leu Gly Ala Leu Gln Ser Leu Leu Gly Thr Gln Leu
                100                    105                    110

Pro Pro Gln Gly Arg Thr Thr Ala His Lys Asp Pro Asn Ala Ile Phe
                115                    120                    125

Leu Ser Phe Gln His Leu Leu Arg Gly Lys Val Arg Phe Leu Met Leu
                130                    135                    140

Val Gly Gly Ser Thr Leu Cys Val Arg Arg Ala Pro Thr Tyr Arg Ala
145                    150                    155                    160

Ser Ser Leu Pro Gln Ser Phe Leu Leu Lys Ser Leu Glu Gln Val Arg
                165                    170                    175

Lys Ile Gln Gly Asp Gly Ala Ala Leu Gln Glu Lys Leu Cys Ala Thr
                180                    185                    190

Tyr Lys Leu Cys His Pro Glu Glu Leu Val Leu Leu Gly His Ser Leu
                195                    200                    205

Gly Ile Pro Trp Ala Pro Leu Ser Ser Cys Pro Ser Gln Ala Leu Gln
                210                    215                    220

Leu Ala Gly Cys Leu Ser Gln Leu His Ser Gly Leu Phe Leu Tyr Gln
225                    230                    235                    240

Gly Leu Leu Gln Ala Leu Glu Gly Ile Ser Pro Glu Leu Gly Pro Thr
                245                    250                    255

Leu Asp Thr Leu Gln Leu Asp Val Ala Asp Phe Ala Thr Thr Ile Trp
                260                    265                    270

Gln Gln Met Glu Glu Leu Gly Met Ala Pro Ala Leu Gln Pro Thr Gln
                275                    280                    285

Gly Ala Met Pro Ala Phe Ala Ser Ala Phe Gln Arg Arg Ala Gly Gly
                290                    295                    300

```
Val Leu Val Ala Ser His Leu Gln Ser Phe Leu Glu Val Ser Tyr Arg
305                 310             315                 320

Val Leu Arg His Leu Ala Gln Pro
                325
```

(2) INFORMATION FOR SEQ ID NO:2:

    (i) SEQUENCE CHARACTERISTICS:

        (A) LENGTH: 340 amino acids
        (B) TYPE: amino acid
        (C) STRANDEDNESS: single
        (D) TOPOLOGY: linear

    (ii) MOLECULE TYPE: peptide

    (vi) ORIGINAL SOURCE

        (A) ORGANISM: human (Homo sapiens)

    (ix) FEATURE

        (A) NAME/KEY:
        (B) LOCATION: 1..153

        (A) NAME/KEY:
        (B) LOCATION: 167..340

    (xi) SEQUENCE DESCRIPTION: SEQ ID NO:2:

```
Ser Pro Ala Pro Pro Ala Cys Asp Leu Arg Val Leu Ser Lys Leu Leu
1               5               10                  15

Arg Asp Ser His Val Leu His Ser Arg Leu Ser Gln Cys Pro Glu Val
            20              25              30

His Pro Leu Pro Thr Pro Val Leu Leu Pro Ala Val Asp Phe Ser Leu
            35              40              45

Gly Glu Trp Lys Thr Gln Met Glu Glu Thr Lys Ala Gln Asp Ile Leu
            50              55              60
```

Gly Ala Val Thr Leu Leu Leu Glu Gly Val Met Ala Ala Arg Gly Gln
65          70          75          80

Leu Gly Pro Thr Cys Leu Ser Ser Leu Leu Gly Gln Leu Ser Gly Gln
                85          90          95

Val Arg Leu Leu Leu Gly Ala Leu Gln Ser Leu Leu Gly Thr Gln Leu
            100         105         110

Pro Pro Gln Gly Arg Thr Thr Ala His Lys Asp Pro Asn Ala Ile Phe
        115         120         125

Leu Ser Phe Gln His Leu Leu Arg Gly Lys Val Arg Phe Leu Met Leu
    130         135         140

Val Gly Gly Ser Thr Leu Cys Val Arg Gly Gly Gly Ser Gly Gly Gly
145         150         155         160

Ser Gly Gly Gly Ser Arg Ala Pro Thr Tyr Arg Ala Ser Ser Leu Pro
            165         170         175

Gln Ser Phe Leu Leu Lys Ser Leu Glu Gln Val Arg Lys Ile Gln Gly
        180         185         190

Asp Gly Ala Ala Leu Gln Glu Lys Leu Cys Ala Thr Tyr Lys Leu Cys
        195         200         205

His Pro Glu Glu Leu Val Leu Leu Gly His Ser Leu Gly Ile Pro Trp
    210         215         220

Ala Pro Leu Ser Ser Cys Pro Ser Gln Ala Leu Gln Leu Ala Gly Cys
225         230         235         240

Leu Ser Gln Leu His Ser Gly Leu Phe Leu Tyr Gln Gly Leu Leu Gln
            245         250         255

Ala Leu Glu Gly Ile Ser Pro Glu Leu Gly Pro Thr Leu Asp Thr Leu
            260         265         270

```
Gln Leu Asp Val Ala Asp Phe Ala Thr Thr Ile Trp Gln Gln Met Glu
        275             280             285

Glu Leu Gly Met Ala Pro Ala Leu Gln Pro Thr Gln Gly Ala Met Pro
    290             295             300

Ala Phe Ala Ser Ala Phe Gln Arg Arg Ala Gly Gly Val Leu Val Ala
305             310             315             320

Ser His Leu Gln Ser Phe Leu Glu Val Ser Tyr Arg Val Leu Arg His
            325             330             335

Leu Ala Gln Pro
            340
```

(2) INFORMATION FOR SEQ ID NO:3:

    (i) SEQUENCE CHARACTERISTICS:

        (A) LENGTH: 344 amino acids
        (B) TYPE: amino acid
        (C) STRANDEDNESS: single
        (D) TOPOLOGY: linear

    (ii) MOLECULE TYPE: peptide

    (vi) ORIGINAL SOURCE

        (A) ORGANISM: human (Homo sapiens)

    (ix) FEATURE

        (A) NAME/KEY:
        (B) LOCATION: 1..153

        (A) NAME/KEY:
        (B) LOCATION: 171..344

    (xi) SEQUENCE DESCRIPTION: SEQ ID NO:3:

```
Ser Pro Ala Pro Pro Ala Cys Asp Leu Arg Val Leu Ser Lys Leu Leu
1               5                   10                  15

Arg Asp Ser His Val Leu His Ser Arg Leu Ser Gln Cys Pro Glu Val
            20                  25                  30

His Pro Leu Pro Thr Pro Val Leu Leu Pro Ala Val Asp Phe Ser Leu
        35                  40                  45

Gly Glu Trp Lys Thr Gln Met Glu Glu Thr Lys Ala Gln Asp Ile Leu
    50                  55                  60

Gly Ala Val Thr Leu Leu Leu Glu Gly Val Met Ala Ala Arg Gly Gln
65              70                  75                  80

Leu Gly Pro Thr Cys Leu Ser Ser Leu Leu Gly Gln Leu Ser Gly Gln
            85                  90                  95

Val Arg Leu Leu Leu Gly Ala Leu Gln Ser Leu Leu Gly Thr Gln Leu
            100                 105                 110

Pro Pro Gln Gly Arg Thr Thr Ala His Lys Asp Pro Asn Ala Ile Phe
        115                 120                 125

Leu Ser Phe Gln His Leu Leu Arg Gly Lys Val Arg Phe Leu Met Leu
    130                 135                 140

Val Gly Gly Ser Thr Leu Cys Val Arg Ser Gly Gly Gly Ser Gly Gly
145                 150                 155                 160

Gly Ser Gly Gly Gly Ser Gly Gly Gly Arg Ala Pro Thr Tyr Arg Ala
            165                 170                 175

Ser Ser Leu Pro Gln Ser Phe Leu Leu Lys Ser Leu Glu Gln Val Arg
            180                 185                 190
```

```
Lys Ile Gln Gly Asp Gly Ala Ala Leu Gln Glu Lys Leu Cys Ala Thr
        195                 200                 205

Tyr Lys Leu Cys His Pro Glu Glu Leu Val Leu Leu Gly His Ser Leu
        210                 215                 220

Gly Ile Pro Trp Ala Pro Leu Ser Ser Cys Pro Ser Gln Ala Leu Gln
225                 230                 235                 240

Leu Ala Gly Cys Leu Ser Gln Leu His Ser Gly Leu Phe Leu Tyr Gln
                245                 250                 255

Gly Leu Leu Gln Ala Leu Glu Gly Ile Ser Pro Glu Leu Gly Pro Thr
                260                 265                 270

Leu Asp Thr Leu Gln Leu Asp Val Ala Asp Phe Ala Thr Thr Ile Trp
        275                 280                 285

Gln Gln Met Glu Glu Leu Gly Met Ala Pro Ala Leu Gln Pro Thr Gln
        290                 295                 300

Gly Ala Met Pro Ala Phe Ala Ser Ala Phe Gln Arg Arg Ala Gly Gly
305                 310                 315                 320

Val Leu Val Ala Ser His Leu Gln Ser Phe Leu Glu Val Ser Tyr Arg
                325                 330                 335

Val Leu Arg His Leu Ala Gln Pro
                340
```

(2) INFORMATION FOR SEQ ID NO:4:

    (i) SEQUENCE CHARACTERISTICS:

        (A) LENGTH: 1047 base pairs
        (B) TYPE: nucleic acid
        (C) STRANDEDNESS: double
        (D) TOPOLOGY: linear

    (ii) MOLECULE TYPE: other nucleic acid, synthetic DNA

    (vi) ORIGINAL SOURCE

        (A) ORGANISM: human (Homo sapiens)

(ix) FEATURE:

    (A) NAME/KEY: sig peptide
    (B) LOCATION: 1..63

    (A) NAM/KEY: CDS
    (B) LOCATION: 64..1047

(xi) SEQUENCE DESCRIPTION: SEQ ID NO:4:

```
ATG GAG CTG ACT GAA TTG CTC CTC GTG GTC ATG CTT CTC CTA ACT GCA    48
Met Glu Leu Thr Glu Leu Leu Leu Val Val Met Leu Leu Leu Thr Ala
    -20              -15              -10

AGG CTA ACG CTG TCC AGC CCG GCT CCT CCT GCT TGT GAC CTC CGA GTC    96
Arg Leu Thr Leu Ser Ser Pro Ala Pro Pro Ala Cys Asp Leu Arg Val
    -5               1                5                10

CTC AGT AAA CTG CTT CGT GAC TCC CAT GTC CTT CAC AGC AGA CTG AGC   144
Leu Ser Lys Leu Leu Arg Asp Ser His Val Leu His Ser Arg Leu Ser
            15               20               25

CAG TGC CCA GAG GTT CAC CCT TTG CCT ACA CCT GTC CTG CTG CCT GCT   192
Gln Cys Pro Glu Val His Pro Leu Pro Thr Pro Val Leu Leu Pro Ala
            30               35               40

GTG GAC TTT AGC TTG GGA GAA TGG AAA ACC CAG ATG GAG GAG ACC AAG   240
Val Asp Phe Ser Leu Gly Glu Trp Lys Thr Gln Met Glu Glu Thr Lys
        45               50               55

GCA CAG GAC ATT CTG GGA GCA GTG ACC CTT CTG CTG GAG GGA GTG ATG   288
Ala Gln Asp Ile Leu Gly Ala Val Thr Leu Leu Leu Glu Gly Val Met
    60               65               70               75
```

```
GCA GCA CGG GGA CAA CTG GGA CCC ACT TGC CTC TCA TCC CTC CTG GGG   336
Ala Ala Arg Gly Gln Leu Gly Pro Thr Cys Leu Ser Ser Leu Leu Gly
                    80              85                  90

CAG CTT TCT GGA CAG GTC CGT CTC CTC CTT GGG GCC CTG CAG AGC CTC   384
Gln Leu Ser Gly Gln Val Arg Leu Leu Leu Gly Ala Leu Gln Ser Leu
                95                  100                 105

CTT GGA ACC CAG CTT CCT CCA CAG GGC AGG ACC ACA GCT CAC AAG GAT   432
Leu Gly Thr Gln Leu Pro Pro Gln Gly Arg Thr Thr Ala His Lys Asp
            110                 115                 120

CCC AAT GCC ATC TTC CTG AGC TTC CAA CAC CTG CTC CGA GGA AAG GTG   480
Pro Asn Ala Ile Phe Leu Ser Phe Gln His Leu Leu Arg Gly Lys Val
            125                 130                 135

CGT TTC CTG ATG CTT GTA GGA GGG TCC ACC CTC TGC GTA CGG CGG GCG   528
Arg Phe Leu Met Leu Val Gly Gly Ser Thr Leu Cys Val Arg Arg Ala
140             145                 150                 155

CCA ACA TAT CGC GCC TCG AGT CTA CCA CAG AGC TTC CTT TTA AAA AGC   576
Pro Thr Tyr Arg Ala Ser Ser Leu Pro Gln Ser Phe Leu Leu Lys Ser
            160                 165                 170

TTA GAG CAA GTG AGG AAG ATC CAG GGC GAT GGC GCA GCG CTC CAG GAG   624
Leu Glu Gln Val Arg Lys Ile Gln Gly Asp Gly Ala Ala Leu Gln Glu
            175                 180                 185

AAG CTG TGT GCC ACC TAC AAG CTG TGC CAC CCC GAG GAG CTG GTG CTG   672
Lys Leu Cys Ala Thr Tyr Lys Leu Cys His Pro Glu Glu Leu Val Leu
            190                 195                 200

CTC GGA CAC TCT CTG GGC ATC CCC TGG GCT CCC CTG AGC AGC TGC CCC   720
Leu Gly His Ser Leu Gly Ile Pro Trp Ala Pro Leu Ser Ser Cys Pro
            205                 210                 215
```

29

```
AGC CAG GCC CTG CAG CTG GCA GGC TGC TTG AGC CAA CTC CAT AGC GGC  768
Ser Gln Ala Leu Gln Leu Ala Gly Cys Leu Ser Gln Leu His Ser Gly
220             225             230             235

CTT TTC CTC TAC CAG GGG CTC CTG CAG GCC CTG GAA GGG ATC TCC CCC  816
Leu Phe Leu Tyr Gln Gly Leu Leu Gln Ala Leu Glu Gly Ile Ser Pro
            240             245             250

GAG TTG GGT CCC ACC TTG GAC ACA CTG CAG CTG GAC GTC GCC GAC TTT  864
Glu Leu Gly Pro Thr Leu Asp Thr Leu Gln Leu Asp Val Ala Asp Phe
            255             260             265

GCC ACC ACC ATC TGG CAG CAG ATG GAA GAA CTG GGA ATG GCC CCT GCC  912
Ala Thr Thr Ile Trp Gln Gln Met Glu Glu Leu Gly Met Ala Pro Ala
        270             275             280

CTG CAG CCC ACC CAG GGT GCC ATG CCG GCC TTC GCC TCT GCT TTC CAG  960
Leu Gln Pro Thr Gln Gly Ala Met Pro Ala Phe Ala Ser Ala Phe Gln
    285             290             295

CGC CGG GCA GGA GGG GTC CTA GTT GCC TCC CAT CTG CAG AGC TTC CTG 1008
Arg Arg Ala Gly Gly Val Leu Val Ala Ser His Leu Gln Ser Phe Leu
300             305             310             315

GAG GTG TCG TAC CGC GTT CTA CGC CAC CTT GCC CAG CCC                1047
Glu Val Ser Tyr Arg Val Leu Arg His Leu Ala Gln Pro
            320             325
```

(2) INFORMATION FOR SEQ ID NO:5:

    (i) SEQUENCE CHARACTERISTICS:

        (A) LENGTH: 1083 base pairs
        (B) TYPE: nucleic acid
        (C) STRANDEDNESS: double
        (D) TOPOLOGY: linear

    (ii) MOLECULE TYPE: other nucleic acid, synthetic DNA

    (vi) ORIGINAL SOURCE

        (A) ORGANISM: human (Homo sapiens)

    (ix) FEATURE:

        (A) NAME/KEY: sig peptide
        (B) LOCATION: 1..63

(A) NAME/KEY: CDS
(B) LOCATION: 64..1083

(xi) SEQUENCE DESCRIPTION: SEQ ID NO:5:

```
ATG GAG CTG ACT GAA TTG CTC CTC GTG GTC ATG CTT CTC CTA ACT GCA    48
Met Glu Leu Thr Glu Leu Leu Leu Val Val Met Leu Leu Leu Thr Ala
    -20              -15              -10

AGG CTA ACG CTG TCC AGC CCG GCT CCT CCT GCT TGT GAC CTC CGA GTC    96
Arg Leu Thr Leu Ser Ser Pro Ala Pro Pro Ala Cys Asp Leu Arg Val
    -5               1                5                     10

CTC AGT AAA CTG CTT CGT GAC TCC CAT GTC CTT CAC AGC AGA CTG AGC   144
Leu Ser Lys Leu Leu Arg Asp Ser His Val Leu His Ser Arg Leu Ser
            15                   20                   25

CAG TGC CCA GAG GTT CAC CCT TTG CCT ACA CCT GTC CTG CTG CCT GCT   192
Gln Cys Pro Glu Val His Pro Leu Pro Thr Pro Val Leu Leu Pro Ala
        30                   35                   40

GTG GAC TTT AGC TTG GGA GAA TGG AAA ACC CAG ATG GAG GAG ACC AAG   240
Val Asp Phe Ser Leu Gly Glu Trp Lys Thr Gln Met Glu Glu Thr Lys
        45                   50                   55

GCA CAG GAC ATT CTG GGA GCA GTG ACC CTT CTG CTG GAG GGA GTG ATG   288
Ala Gln Asp Ile Leu Gly Ala Val Thr Leu Leu Leu Glu Gly Val Met
60               65                   70                       75

GCA GCA CGG GGA CAA CTG GGA CCC ACT TGC CTC TCA TCC CTC CTG GGG   336
Ala Ala Arg Gly Gln Leu Gly Pro Thr Cys Leu Ser Ser Leu Leu Gly
                80                   85                   90
```

```
CAG CTT TCT GGA CAG GTC CGT CTC CTC CTT GGG GCC CTG CAG AGC CTC    384
Gln Leu Ser Gly Gln Val Arg Leu Leu Leu Gly Ala Leu Gln Ser Leu
            95                  100             105

CTT GGA ACC CAG CTT CCT CCA CAG GGC AGG ACC ACA GCT CAC AAG GAT    432
Leu Gly Thr Gln Leu Pro Pro Gln Gly Arg Thr Thr Ala His Lys Asp
            110                 115             120

CCC AAT GCC ATC TTC CTG AGC TTC CAA CAC CTG CTC CGA GGA AAG GTG    480
Pro Asn Ala Ile Phe Leu Ser Phe Gln His Leu Leu Arg Gly Lys Val
            125                 130             135

CGT TTC CTG ATG CTT GTA GGA GGG TCC ACC CTC TGC GTC AGG GGT GGC    528
Arg Phe Leu Met Leu Val Gly Gly Ser Thr Leu Cys Val Arg Gly Gly
140                 145             150                 155

GGT TCT GGA GGT GGT TCC GGA GGG GGT TCT AGA GCA CCA ACA TAT CGC    576
Gly Ser Gly Gly Gly Ser Gly Gly Gly Ser Arg Ala Pro Thr Tyr Arg
                160                 165             170

GCC TCG AGT CTA CCA CAG AGC TTC CTT TTA AAA AGC TTA GAG CAA GTG    624
Ala Ser Ser Leu Pro Gln Ser Phe Leu Leu Lys Ser Leu Glu Gln Val
            175                 180             185

AGG AAG ATC CAG GGC GAT GGC GCA GCG CTC CAG GAG AAG CTG TGT GCC    672
Arg Lys Ile Gln Gly Asp Gly Ala Ala Leu Gln Glu Lys Leu Cys Ala
            190                 195             200

ACC TAC AAG CTG TGC CAC CCC GAG GAG CTG GTG CTG CTC GGA CAC TCT    720
Thr Tyr Lys Leu Cys His Pro Glu Glu Leu Val Leu Leu Gly His Ser
            205                 210             215

CTG GGC ATC CCC TGG GCT CCC CTG AGC AGC TGC CCC AGC CAG GCC CTG    768
Leu Gly Ile Pro Trp Ala Pro Leu Ser Ser Cys Pro Ser Gln Ala Leu
220                 225             230                 235
```

```
CAG CTG GCA GGC TGC TTG AGC CAA CTC CAT AGC GGC CTT TTC CTC TAC 816
Gln Leu Ala Gly Cys Leu Ser Gln Leu His Ser Gly Leu Phe Leu Tyr
            240                 245                 250

CAG GGG CTC CTG CAG GCC CTG GAA GGG ATC TCC CCC GAG TTG GGT CCC 864
Gln Gly Leu Leu Gln Ala Leu Glu Gly Ile Ser Pro Glu Leu Gly Pro
            255                 260                 265

ACC TTG GAC ACA CTG CAG CTG GAC GTC GCC GAC TTT GCC ACC ACC ATC 912
Thr Leu Asp Thr Leu Gln Leu Asp Val Ala Asp Phe Ala Thr Thr Ile
            270                 275                 280

TGG CAG CAG ATG GAA GAA CTG GGA ATG GCC CCT GCC CTG CAG CCC ACC 960
Trp Gln Gln Met Glu Glu Leu Gly Met Ala Pro Ala Leu Gln Pro Thr
            285                 290                 295

CAG GGT GCC ATG CCG GCC TTC GCC TCT GCT TTC CAG CGC CGG GCA GGA 1008
Gln Gly Ala Met Pro Ala Phe Ala Ser Ala Phe Gln Arg Arg Ala Gly
300                 305                 310                 315

GGG GTC CTA GTT GCC TCC CAT CTG CAG AGC TTC CTG GAG GTG TCG TAC 1056
Gly Val Leu Val Ala Ser His Leu Gln Ser Phe Leu Glu Val Ser Tyr
            320                 325                 330

CGC GTT CTA CGC CAC CTT GCC CAG CCC                             1083
Arg Val Leu Arg His Leu Ala Gln Pro
            335                 340
```

(2) INFORMATION FOR SEQ ID NO:6:

    (i) SEQUENCE CHARACTERISTICS:

        (A) LENGTH: 1095 base pairs
        (B) TYPE: nucleic acid
        (C) STRANDEDNESS: double
        (D) TOPOLOGY: linear

    (ii) MOLECULE TYPE: other nucleic acid, synthetic DNA

    (vi) ORIGINAL SOURCE

        (A) ORGANISM: human (Homo sapiens)

    (ix) FEATURE:

        (A) NAME/KEY: sig peptide
        (B) LOCATION: 1..63

(A) NAME/KEY: CDS
(B) LOCATION: 64..1095

(xi) SEQUENCE DESCRIPTION: SEQ ID NO:6:


ATG GAG CTG ACT GAA TTG CTC CTC GTG GTC ATG CTT CTC CTA ACT GCA  48
Met Glu Leu Thr Glu Leu Leu Leu Val Val Met Leu Leu Leu Thr Ala
    -20             -15             -10


AGG CTA ACG CTG TCC AGC CCG GCT CCT CCT GCT TGT GAC CTC CGA GTC  96
Arg Leu Thr Leu Ser Ser Pro Ala Pro Pro Ala Cys Asp Leu Arg Val
    -5              1               5                   10


CTC AGT AAA CTG CTT CGT GAC TCC CAT GTC CTT CAC AGC AGA CTG AGC  144
Leu Ser Lys Leu Leu Arg Asp Ser His Val Leu His Ser Arg Leu Ser
            15              20              25


CAG TGC CCA GAG GTT CAC CCT TTG CCT ACA CCT GTC CTG CTG CCT GCT  192
Gln Cys Pro Glu Val His Pro Leu Pro Thr Pro Val Leu Leu Pro Ala
        30              35              40


GTG GAC TTT AGC TTG GGA GAA TGG AAA ACC CAG ATG GAG GAG ACC AAG  240
Val Asp Phe Ser Leu Gly Glu Trp Lys Thr Gln Met Glu Glu Thr Lys
        45              50              55


GCA CAG GAC ATT CTG GGA GCA GTG ACC CTT CTG CTG GAG GGA GTG ATG  288
Ala Gln Asp Ile Leu Gly Ala Val Thr Leu Leu Leu Glu Gly Val Met
    60              65              70              75

```
GCA GCA CGG GGA CAA CTG GGA CCC AAT TGC CTC TCA TCC CTC CTG GGG   336
Ala Ala Arg Gly Gln Leu Gly Pro Thr Cys Leu Ser Ser Leu Leu Gly
                80                  85                  90


CAG CTT TCT GGA CAG GTC CGT CTC CTC CTT GGG GCC CTG CAG AGC CTC   384
Gln Leu Ser Gly Gln Val Arg Leu Leu Leu Gly Ala Leu Gln Ser Leu
                95                 100                 105


CTT GGA ACC CAG CTT CCT CCA CAG GGC AGG ACC ACA GCT CAC AAG GAT   432
Leu Gly Thr Gln Leu Pro Pro Gln Gly Arg Thr Thr Ala His Lys Asp
               110                 115                 120


CCC AAT GCC ATC TTC CTG AGC TTC CAA CAC CTG CTC CGA GGA AAG GTG   480
Pro Asn Ala Ile Phe Leu Ser Phe Gln His Leu Leu Arg Gly Lys Val
               125                 130                 135


CGT TTC CTG ATG CTT GTA GGA GGG TCC ACC CTC TGC GTA CGG TCC GGA   528
Arg Phe Leu Met Leu Val Gly Gly Ser Thr Leu Cys Val Arg Ser Gly
140                 145                 150                 155


GGT GGC TCT GGC GGT GGT TCT GGT GGC GGC TCC GGA GGC GGT CGT GCG   576
Gly Gly Ser Gly Gly Gly Ser Gly Gly Gly Ser Gly Gly Gly Arg Ala
               160                 165                 170


CCA ACA TAT CGC GCC TCG AGT CTA CCA CAG AGC TTC CTT TTA AAA AGC   624
Pro Thr Tyr Arg Ala Ser Ser Leu Pro Gln Ser Phe Leu Leu Lys Ser
               175                 180                 185


TTA GAG CAA GTG AGG AAG ATC CAG GGC GAT GGC GCA GCG CTC CAG GAG   672
Leu Glu Gln Val Arg Lys Ile Gln Gly Asp Gly Ala Ala Leu Gln Glu
               190                 195                 200


AAG CTG TGT GCC ACC TAC AAG CTG TGC CAC CCC GAG GAG CTG GTG CTG   720
Lys Leu Cys Ala Thr Tyr Lys Leu Cys His Pro Glu Glu Leu Val Leu
               205                 210                 215


CTC GGA CAC TCT CTG GGC ATC CCC TGG GCT CCC CTG AGC AGC TGC CCC   768
Leu Gly His Ser Leu Gly Ile Pro Trp Ala Pro Leu Ser Ser Cys Pro
220                 225                 230                 235
```

```
AGC CAG GCC CTG CAG CTG GCA GGC TGC TTG AGC CAA CTC CAT AGC GGC  816
Ser Gln Ala Leu Gln Leu Ala Gly Cys Leu Ser Gln Leu His Ser Gly
                240                 245                 250

CTT TTC CTC TAC CAG GGG CTC CTG CAG GCC CTG GAA GGG ATC TCC CCC  864
Leu Phe Leu Tyr Gln Gly Leu Leu Gln Ala Leu Glu Gly Ile Ser Pro
                255                 260                 265

GAG TTG GGT CCC ACC TTG GAC ACA CTG CAG CTG GAC GTC GCC GAC TTT  912
Glu Leu Gly Pro Thr Leu Asp Thr Leu Gln Leu Asp Val Ala Asp Phe
                270                 275                 280

GCC ACC ACC ATC TGG CAG CAG ATG GAA GAA CTG GGA ATG GCC CCT GCC  960
Ala Thr Thr Ile Trp Gln Gln Met Glu Glu Leu Gly Met Ala Pro Ala
                285                 290                 295

CTG CAG CCC ACC CAG GGT GCC ATG CCG GCC TTC GCC TCT GCT TTC CAG 1008
Leu Gln Pro Thr Gln Gly Ala Met Pro Ala Phe Ala Ser Ala Phe Gln
300                 305                 310                 315

CGC CGG GCA GGA GGG GTC CTA GTT GCC TCC CAT CTG CAG AGC TTC CTG 1056
Arg Arg Ala Gly Gly Val Leu Val Ala Ser His Leu Gln Ser Phe Leu
                320                 325                 330

GAG GTG TCG TAC CGC GTT CTA CGC CAC CTT GCC CAG CCC                1095
Glu Val Ser Tyr Arg Val Leu Arg His Leu Ala Gln Pro
                335                 340
```

(2) INFORMATION FOR SEQ ID NO:7:

    (i) SEQUENCE CHARACTERISTICS:

        (A) LENGTH: 44 bases
        (B) TYPE: nucleic acid
        (C) STRANDEDNESS: single
        (D) TOPOLOGY: linear

    (ii) MOLECULE TYPE: other nucleic acid, synthetic DNA

    (ix) FEATURE:

        (A) NAME/KEY: sig peptide
        (B) LOCATION: 27..44

    (xi) SEQUENCE DESCRIPTION: SEQ ID NO:7:

        CTCTCCAAGC TTGAATTCCG GCCAGAATGG AGCTGACTGA ATTG      44

(2) INFORMATION FOR SEQ ID NO:8:

(i) SEQUENCE CHARACTERISTICS:

    (A) LENGTH: 47 bases
    (B) TYPE: nucleic acid
    (C) STRANDEDNESS: single
    (D) TOPOLOGY: linear

(ii) MOLECULE TYPE: other nucleic acid, synthetic DNA

(ix) FEATURE:

    (A) NAME/KEY: CDS
    (B) LOCATION: 23..47

(xi) SEQUENCE DESCRIPTION: SEQ ID NO:8:

    GTAGAGGTAC CGCGGCCGCT TACCCTTCCT GAGACAGATT CTGGGAG       47

(2) INFORMATION FOR SEQ ID NO:9:

(i) SEQUENCE CHARACTERISTICS:

    (A) LENGTH: 24 bases
    (B) TYPE: nucleic acid
    (C) STRANDEDNESS: single
    (D) TOPOLOGY: linear

(ii) MOLECULE TYPE: other nucleic acid, synthetic DNA

(ix) FEATURE:

    (A) NAME/KEY: CDS
    (B) LOCATION: 1..24

(xi) SEQUENCE DESCRIPTION: SEQ ID NO:9:

    TGAACCTCTG GGCACTGGCT CAGT       24

(2) INFORMATION FOR SEQ ID NO:10:

(i) SEQUENCE CHARACTERISTICS:

    (A) LENGTH: 24 bases
    (B) TYPE: nucleic acid
    (C) STRANDEDNESS: single
    (D) TOPOLOGY: linear

(ii) MOLECULE TYPE: other nucleic acid, synthetic DNA

(ix) FEATURE:

    (A) NAME/KEY: CDS
    (B) LOCATION: 1.. 24

(xi) SEQUENCE DESCRIPTION: SEQ ID NO:10:

    GCTGCCTGCT GTGGACTTTA GCTT       24

(2) INFORMATION FOR SEQ ID NO:11:

(i) SEQUENCE CHARACTERISTICS:

(A) LENGTH: 24 bases
(B) TYPE: nucleic acid
(C) STRANDEDNESS: single
(D) TOPOLOGY: linear

(ii) MOLECULE TYPE: other nucleic acid, synthetic DNA

(ix) FEATURE:

(A) NAME/KEY: CDS
(B) LOCATION: 1..24

(xi) SEQUENCE DESCRIPTION: SEQ ID NO:11:

TGTTGGAAGC TCAGGAAGAT GGCA          24

(2) INFORMATION FOR SEQ ID NO:12:

(i) SEQUENCE CHARACTERISTICS:

(A) LENGTH: 24 bases
(B) TYPE: nucleic acid
(C) STRANDEDNESS: single
(D) TOPOLOGY: linear

(ii) MOLECULE TYPE: other nucleic acid, synthetic DNA

(ix) FEATURE:

(A) NAME/KEY: CDS
(B) LOCATION: 1..24

(xi) SEQUENCE DESCRIPTION: SEQ ID NO:12:

CCTGATGCTT GTAGGAGGGT CCAC          24

(2) INFORMATION FOR SEQ ID NO:13:

(i) SEQUENCE CHARACTERISTICS:

(A) LENGTH: 24 bases
(B) TYPE: nucleic acid
(C) STRANDEDNESS: single
(D) TOPOLOGY: linear

(ii) MOLECULE TYPE: other nucleic acid, synthetic DNA

(ix) FEATURE:

(A) NAME/KEY: CDS
(B) LOCATION: 1..24

(xi) SEQUENCE DESCRIPTION: SEQ ID NO:13:

TCAAGAGTTC GTGTATCCTG TTCA        24

(2) INFORMATION FOR SEQ ID NO:14:

    (i) SEQUENCE CHARACTERISTICS:

        (A) LENGTH: 24 bases
        (B) TYPE: nucleic acid
        (C) STRANDEDNESS: single
        (D) TOPOLOGY: linear

    (ii) MOLECULE TYPE: other nucleic acid, synthetic DNA

    (ix) FEATURE:

        (A) NAME/KEY: CDS
        (B) LOCATION: 1..24

    (xi) SEQUENCE DESCRIPTION: SEQ ID NO:14:

        GAATGGAACT CGTGGACTCT TTCC        24

(2) INFORMATION FOR SEQ ID NO:15:

    (i) SEQUENCE CHARACTERISTICS:

        (A) LENGTH: 17 bases
        (B) TYPE: nucleic acid
        (C) STRANDEDNESS: single
        (D) TOPOLOGY: linear

    (ii) MOLECULE TYPE: other nucleic acid, synthetic DNA

    (xi) SEQUENCE DESCRIPTION: SEQ ID NO:15:

        GTAAAACGAC GGCCAGT        17

(2) INFORMATION FOR SEQ ID NO:16:

    (i) SEQUENCE CHARACTERISTICS:

        (A) LENGTH: 17 bases
        (B) TYPE: nucleic acid
        (C) STRANDEDNESS: single
        (D) TOPOLOGY: linear

    (ii) MOLECULE TYPE: other nucleic acid, synthetic DNA

    (xi) SEQUENCE DESCRIPTION: SEQ ID NO:16:

        CAGGAAACAG CTATGAC        17

(2) INFORMATION FOR SEQ ID NO:17:

    (i) SEQUENCE CHARACTERISTICS:

        (A) LENGTH: 13 amino acids
        (B) TYPE: amino acid

(C) STRANDEDNESS: single
(D) TOPOLOGY: linear

(ii) MOLECULE TYPE: peptide

(xi) SEQUENCE DESCRIPTION: SEQ ID NO:17:

```
        Gly Gly Gly Ser Gly Gly Gly Ser Gly Gly Gly Ser Arg
          1               5                       10
```

(2) INFORMATION FOR SEQ ID NO:18:

(i) SEQUENCE CHARACTERISTICS:

(A) LENGTH: 66 bases
(B) TYPE: nucleic acid
(C) STRANDEDNESS: single
(D) TOPOLOGY: linear

(ii) MOLECULE TYPE: other nucleic acid, synthetic DNA

(ix) FEATURE:

(A) NAME/KEY: CDS
(B) LOCATION: 1..3
(A) NAME/KEY: CDS
(B) LOCATION: 43..66

(xi) SEQUENCE DESCRIPTION: SEQ ID NO:18:

```
TGCTCTAGAA CCGCCTCCGG AACCACCTCC AGAACCGCCA CCCCTGACGC AGAGGGTGGA 60
CCCTCC                                                            66
```

(2) INFORMATION FOR SEQ ID NO:19:

(i) SEQUENCE CHARACTERISTICS:

(A) LENGTH: 45 bases
(B) TYPE: nucleic acid
(C) STRANDEDNESS: single
(D) TOPOLOGY: linear

(ii) MOLECULE TYPE: other nucleic acid, synthetic DNA

(ix) FEATURE:

(A) NAME/KEY: CDS
(B) LOCATION: 22..45

(xi) SEQUENCE DESCRIPTION: SEQ ID NO:19:

```
GGTTCCGGAG GCGGTTCTAG AGCACCAACA TATCGCGCCT CGAGT          45
```

(2) INFORMATION FOR SEQ ID NO:20:

    (i) SEQUENCE CHARACTERISTICS:

        (A) LENGTH: 48 bases
        (B) TYPE: nucleic acid
        (C) STRANDEDNESS: single
        (D) TOPOLOGY: linear

    (ii) MOLECULE TYPE: other nucleic acid, synthetic DNA

    (ix) FEATURE:

        (A) NAME/KEY: CDS
        (B) LOCATION: 22..48

    (xi) SEQUENCE DESCRIPTION: SEQ ID NO:20:

        CATTCCGCGG GGTACCGCGG CCGCTCAGGG CTGGGCAAGG TGGCGTAG      48

(2) INFORMATION FOR SEQ ID NO:21:

    (i) SEQUENCE CHARACTERISTICS:

        (A) LENGTH: 24 bases
        (B) TYPE: nucleic acid
        (C) STRANDEDNESS: single
        (D) TOPOLOGY: linear

    (ii) MOLECULE TYPE: other nucleic acid, synthetic DNA

    (ix) FEATURE:

        (A) NAME/KEY: CDS
        (B) LOCATION: 1..24

    (xi) SEQUENCE DESCRIPTION: SEQ ID NO:21:

        GGCTGCTTGA GCCAACTCCA TAGC      24

(2) INFORMATION FOR SEQ ID NO:22:

    (i) SEQUENCE CHARACTERISTICS:

        (A) LENGTH: 24 bases
        (B) TYPE: nucleic acid
        (C) STRANDEDNESS: single
        (D) TOPOLOGY: linear

    (ii) MOLECULE TYPE: other nucleic acid, synthetic DNA

    (ix) FEATURE:

        (A) NAME/KEY: CDS
        (B) LOCATION: 1..24

    (xi) SEQUENCE DESCRIPTION: SEQ ID NO:22:

GACCCAACTC GGGGGAGATC CCTT      24

(2) INFORMATION FOR SEQ ID NO:23:

    (i) SEQUENCE CHARACTERISTICS:

        (A) LENGTH: 57 bases
        (B) TYPE: nucleic acid
        (C) STRANDEDNESS: single
        (D) TOPOLOGY: linear

    (ii) MOLECULE TYPE: other nucleic acid, synthetic DNA

    (ix) FEATURE:

        (A) NAME/KEY: CDS
        (B) LOCATION: 1..27
        (A) NAME/KEY: CDS
        (B) LOCATION: 28..57
        (A) NAME/KEY: mutation
        (B) LOCATION: 25
        (A) NAME/KEY: mutation
        (B) LOCATION: 33..34

    (xi) SEQUENCE DESCRIPTION: SEQ ID NO:23:

        TAGACTCGAG GCGCGATATG TTGGCGCCCG CCGTACGCAG AGGGTGGACC CTCCTAC      57

(2) INFORMATION FOR SEQ ID NO:24:

    (i) SEQUENCE CHARACTERISTICS:

        (A) LENGTH: 17 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:24:

**Ser Gly Gly Gly Ser Gly Gly Gly Ser Gly Gly Gly Ser Gly Gly Gly Arg**
**1**          **5**          **10**          **15**

(2) INFORMATION FOR SEQ ID NO:25:

    (i) SEQUENCE CHARACTERISTICS:

        (A) LENGTH: 61 bases
        (B) TYPE: nucleic acid
        (C) STRANDEDNESS: single
        (D) TOPOLOGY: linear

    (ii) MOLECULE TYPE: other nucleic acid, synthetic DNA

(ix) FEATURE:

(A) NAME/KEY CDS of TPO
(B) LOCATION: 1..6
(A) NAME/KEY: linker peptide
(B) LOCATION: 7..57
(A) NAME/KEY: CDS of ND28
(B) LOCATION: 58..61
(A) NAME/KEY: SplI
(B) LOCATION: 1..5
(A) NAME/KEY: MroI
(B) LOCATION: 7..12
(A) NAME/KEY: MroI
(B) LOCATION: 43..48
(A) NAME/KEY BbeI
(B) LOCATION: 58..61
(A) NAME/KEY: mutation
(B) LOCATION: 4..5

(xi) SEQUENCE DESCRIPTION: SEQ ID NO:25:


GTACGGTCCG GAGGTGGCTC TGGCGGTGGT TCTGGTGGCG GCTCCGGAGG CGGTCGTGCG 60

C                                                                                                61


(2) INFORMATION FOR SEQ ID NO:26:

(i) SEQUENCE CHARACTERISTICS:

(A) LENGTH: 53 bases
(B) TYPE: nucleic acid
(C) STRANDEDNESS: single
(D) TOPOLOGY: linear

(ii) MOLECULE TYPE: other nucleic acid, synthetic DNA

(ix) FEATURE:

(A) NAME/KEY: CDS of TPO
(B) LOCATION: 52..53
(A) NAME/KEY linker peptide
(B) LOCATION: 1..51
(A) NAME/KEY: SplI
(B) LOCATION: 53
(A) NAME/KEY: MroI
(B) LOCATION: 10..15
(A) NAME/KEY: MroI
(B) LOCATION: 46..51

(xi) SEQUENCE DESCRIPTION: SEQ ID NO:26:

ACGACCGCCT CCGGAGCCGC CACCAGAACC ACCGCCAGAG CCACCTCCGG ACC          53